# EUROPEAN PATENT APPLICATION

(11) **EP 1 118 669 A2**
(43) Date of publication of application: **25.07.2001**
(21) Application number: 00310997.2
(22) Date of filing: 08.12.2000
(51) Int. Cl.: C12N 15/82, A01H 5/00, C07K 1/00

(54) **Production of camelid antibodies in plants**

(30) Priority: 17.12.1999 EP 99310188
(71) Applicant: UNILEVER PLC, London EC4P 4BQ (GB); UNILEVER N.V., 3013 AL Rotterdam (NL)
(72) Inventor: Frenken, Leo Gerard Joseph, 3130 AC Vlaardingen (NL); Van der Logt, Cornelis Paul Erik, 3130 Vlardingen (NL); Jobling, Stephen Alan, Sharnbrook, Bedford, MK44 1LQ (GB); Teh, Yih-Miin, Sharnbrook, Bedford, MK44 1LQ (GB)
(74) Representative: Van Velzen, Maaike Mathilde

(57) **Abstract**

A method for modifying a plant to produce antibodies or active fragments or derivatives thereof in a desired cellular compartment comprising introducing into a plant a DNA sequence encoding a heavy chain immunoglobulin or an active fragment or derivative thereof or a sequence encoding a protein functionally equivalent thereto and plants so modified are disclosed.

SEQUENCE LISTING

<110> Unilever plc

      Unilever NV

<120>  Production of Antibodies

<130>  T7060

<160>  67

<170>  PatentIn version 3.0

<210>  1

<211>  440

<212>  DNA

<213>  Artificial

<220>

<223>   VHH with peptide linker

<220>

<221>  CDS

<222>  (1)..(417)

<400>  1
cag gtg cag ctg cag gag tca ggg gga gga ttg gtg cag gct ggg ggc       48
Gln Val Gln Leu Gln Glu Ser Gly Gly Gly Leu Val Gln Ala Gly Gly
1               5                   10                  15

tct ctg aga ctc tcc tgt gca gcc tcg gga cgc gcc acc agt ggt cat       96
Ser Leu Arg Leu Ser Cys Ala Ala Ser Gly Arg Ala Thr Ser Gly His
            20                  25                  30

ggt cac tat ggt atg ggc tgg ttc cgc cag gtt cca ggg aag gag cgt      144
Gly His Tyr Gly Met Gly Trp Phe Arg Gln Val Pro Gly Lys Glu Arg
        35                  40                  45

gag ttt gtc gca gct att agg tgg agt ggt aaa gag aca tgg tat aaa      192
Glu Phe Val Ala Ala Ile Arg Trp Ser Gly Lys Glu Thr Trp Tyr Lys
    50                  55                  60

gac tcc gtg aag ggc cga ttc acc atc tcc aga gat aac gcc aag act      240
Asp Ser Val Lys Gly Arg Phe Thr Ile Ser Arg Asp Asn Ala Lys Thr
65                  70                  75                  80

acg gtt tat ctg caa atg aac agc ctg aaa cct gaa gat acg gcc gtt      288
Thr Val Tyr Leu Gln Met Asn Ser Leu Lys Pro Glu Asp Thr Ala Val
                85                  90                  95

tat tat tgt gcc gct cga ccg gtc cgc gtg gat gat att tcc ctg ccg      336
Tyr Tyr Cys Ala Ala Arg Pro Val Arg Val Asp Asp Ile Ser Leu Pro
            100                 105                 110

gtt ggg ttt gac tac tgg ggc cag ggg acc cag gtc acc gtc tcc tca      384
Val Gly Phe Asp Tyr Trp Gly Gln Gly Thr Gln Val Thr Val Ser Ser
        115                 120                 125

gaa caa aaa ctc atc tca gaa gag gat ctg aat taataagggc taagctcgaa    437
Glu Gln Lys Leu Ile Ser Glu Glu Asp Leu Asn
    130                 135

ttc                                                                  440

<210>  2

<211>  139

<212>  PRT

<213>  Artificial

<400>  2

Gln Val Gln Leu Gln Glu Ser Gly Gly Gly Leu Val Gln Ala Gly Gly
1               5                   10                  15

Ser Leu Arg Leu Ser Cys Ala Ala Ser Gly Arg Ala Thr Ser Gly His
            20                  25                  30

Gly His Tyr Gly Met Gly Trp Phe Arg Gln Val Pro Gly Lys Glu Arg
        35                  40                  45

Glu Phe Val Ala Ala Ile Arg Trp Ser Gly Lys Glu Thr Trp Tyr Lys
    50                  55                  60

Asp Ser Val Lys Gly Arg Phe Thr Ile Ser Arg Asp Asn Ala Lys Thr
65                  70                  75                  80

Thr Val Tyr Leu Gln Met Asn Ser Leu Lys Pro Glu Asp Thr Ala Val
                85                  90                  95

Tyr Tyr Cys Ala Ala Arg Pro Val Arg Val Asp Asp Ile Ser Leu Pro
            100                 105                 110

Val Gly Phe Asp Tyr Trp Gly Gln Gly Thr Gln Val Thr Val Ser Ser
        115                 120                 125

Glu Gln Lys Leu Ile Ser Glu Glu Asp Leu Asn
    130                 135

<210>  3

<211>  11

<212>  PRT

<213>  Artificial

<220>

<223>  myc linker

<400>  3

Glu Gln Lys Leu Ile Ser Glu Glu Asp Leu Asn
1               5                   10

<210>  4

<211>  471

<212>  DNA

<213>  Artificial

<220>

<223>   VHH with linker

<220>

<221>  CDS

<222>  (1)..(459)

<400>  4
cag gtg cag ctg cag cag tca ggg gga ggc ttg gtg cag gct ggg ggg       48
Gln Val Gln Leu Gln Gln Ser Gly Gly Gly Leu Val Gln Ala Gly Gly
1               5                   10                  15

tct ctg aga ctc tcc tgt gta gct tct gaa agc agc ttc agc aac aat       96
Ser Leu Arg Leu Ser Cys Val Ala Ser Glu Ser Ser Phe Ser Asn Asn
            20                  25                  30

cac atg ggc tgg tac cgc cgg gct cca ggg aac cag cgc gag ctg gtc      144
His Met Gly Trp Tyr Arg Arg Ala Pro Gly Asn Gln Arg Glu Leu Val
        35                  40                  45

gca act att agt cct ggt ggt agc aca cac tat gta gac tcc gtg aag      192
Ala Thr Ile Ser Pro Gly Gly Ser Thr His Tyr Val Asp Ser Val Lys
    50                  55                  60

ggc cga ttc acc atc tcc cga gac aac gcc aag aac aca gtg tat cta      240
Gly Arg Phe Thr Ile Ser Arg Asp Asn Ala Lys Asn Thr Val Tyr Leu
65                  70                  75                  80

caa atg gac agc ctg aaa cca gag gac acg gcc gtc tat tac tgt gct      288
Gln Met Asp Ser Leu Lys Pro Glu Asp Thr Ala Val Tyr Tyr Cys Ala
                85                  90                  95

gcc aag ggg agg ggg ctg cag gct atg cag tac tgg ggc cag ggg acc      336
Ala Lys Gly Arg Gly Leu Gln Ala Met Gln Tyr Trp Gly Gln Gly Thr

            100                 105                 110

ctg gtc acc gtc tcc tca gcg cac cac agc gaa gac ccc agc tcc gcg 384
Leu Val Thr Val Ser Ser Ala His His Ser Glu Asp Pro Ser Ser Ala
        115                 120                 125

gcc gcc cat cac cat cac cat cac ggg gcc gca gaa caa aaa ctc atc 432
Ala Ala His His His His His His Gly Ala Ala Glu Gln Lys Leu Ile
    130                 135                 140

tca gaa gag gat ctg aat ggg gcc gca tagtaacaat tg 471
Ser Glu Glu Asp Leu Asn Gly Ala Ala
145                 150

<210> 5

<211> 153

<212> PRT

<213> Artificial

<400> 5

Gln Val Gln Leu Gln Gln Ser Gly Gly Gly Leu Val Gln Ala Gly Gly
1               5                   10                  15

Ser Leu Arg Leu Ser Cys Val Ala Ser Glu Ser Ser Phe Ser Asn Asn
            20                  25                  30

His Met Gly Trp Tyr Arg Arg Ala Pro Gly Asn Gln Arg Glu Leu Val
        35                  40                  45

Ala Thr Ile Ser Pro Gly Gly Ser Thr His Tyr Val Asp Ser Val Lys
    50                  55                  60

Gly Arg Phe Thr Ile Ser Arg Asp Asn Ala Lys Asn Thr Val Tyr Leu
65                  70                  75                  80

Gln Met Asp Ser Leu Lys Pro Glu Asp Thr Ala Val Tyr Tyr Cys Ala
                85                  90                  95

Ala Lys Gly Arg Gly Leu Gln Ala Met Gln Tyr Trp Gly Gln Gly Thr
            100                 105                 110

Leu Val Thr Val Ser Ser Ala His His Ser Glu Asp Pro Ser Ser Ala
        115                 120                 125

Ala Ala His His His His His His Gly Ala Ala Glu Gln Lys Leu Ile
    130                 135                 140

Ser Glu Glu Asp Leu Asn Gly Ala Ala
145                 150

<210> 6

<211> 468

<212> DNA

<213> Artificial

<220>

<223> VHH with linker

<220>

<221> CDS

<222> (1)..(456)

<400> 6
cag gtg cag ctg cag gag tct ggg gga ggc ctg gtg cag gct ggg ggg 48
Gln Val Gln Leu Gln Glu Ser Gly Gly Gly Leu Val Gln Ala Gly Gly
1               5                   10                  15

tct ctg aga ctc tcc tgt gta gcc tct gga aac acc ttc agt atc ata 96
Ser Leu Arg Leu Ser Cys Val Ala Ser Gly Asn Thr Phe Ser Ile Ile
            20                  25                  30

gct atg gcc tgg tac cgc cag gct cca ggg aag cag cgc gag gtg gtc 144
Ala Met Ala Trp Tyr Arg Gln Ala Pro Gly Lys Gln Arg Glu Val Val
        35                  40                  45

gca agt att aat agt att ggc agc aca aat tat gca gac tcc gtg aag 192
Ala Ser Ile Asn Ser Ile Gly Ser Thr Asn Tyr Ala Asp Ser Val Lys
    50                  55                  60

ggg cga ttc acc atc tcc aga gac aac gcc aag aac aca gtg tat ctg 240
Gly Arg Phe Thr Ile Ser Arg Asp Asn Ala Lys Asn Thr Val Tyr Leu
65                  70                  75                  80

caa atg agc agc ctg aaa cct gag gac acg gcc gtc tat tac tgt gct 288
Gln Met Ser Ser Leu Lys Pro Glu Asp Thr Ala Val Tyr Tyr Cys Ala
                85                  90                  95

gcc ggt aat ttg ctg gtt aag agg cct tac tgg ggc cag ggg acc ctg 336
Ala Gly Asn Leu Leu Val Lys Arg Pro Tyr Trp Gly Gln Gly Thr Leu
            100                 105                 110

gtc acc gtc tcc tca gaa ccc aag aca cca aaa cca caa cca gcg gcc 384
Val Thr Val Ser Ser Glu Pro Lys Thr Pro Lys Pro Gln Pro Ala Ala
        115                 120                 125

gcc cat cac cat cac cat cac ggg gcc gca gaa caa aaa ctc atc tca 432
Ala His His His His His His Gly Ala Ala Glu Gln Lys Leu Ile Ser
    130                 135                 140

gaa gag gat ctg aat ggg gcc gca tagtaacaat tg 468
Glu Glu Asp Leu Asn Gly Ala Ala
145                 150

<210> 7

<211> 152

<212>  PRT

<213>  Artificial

<400>  7

Gln Val Gln Leu Gln Glu Ser Gly Gly Gly Leu Val Gln Ala Gly Gly
1               5                   10                  15

Ser Leu Arg Leu Ser Cys Val Ala Ser Gly Asn Thr Phe Ser Ile Ile
            20                  25                  30

Ala Met Ala Trp Tyr Arg Gln Ala Pro Gly Lys Gln Arg Glu Val Val
        35                  40                  45

Ala Ser Ile Asn Ser Ile Gly Ser Thr Asn Tyr Ala Asp Ser Val Lys
    50                  55                  60

Gly Arg Phe Thr Ile Ser Arg Asp Asn Ala Lys Asn Thr Val Tyr Leu
65                  70                  75                  80

Gln Met Ser Ser Leu Lys Pro Glu Asp Thr Ala Val Tyr Tyr Cys Ala
                85                  90                  95

Ala Gly Asn Leu Leu Val Lys Arg Pro Tyr Trp Gly Gln Gly Thr Leu
            100                 105                 110

Val Thr Val Ser Ser Glu Pro Lys Thr Pro Lys Pro Gln Pro Ala Ala
        115                 120                 125

Ala His His His His His His Gly Ala Ala Glu Gln Lys Leu Ile Ser
    130                 135                 140

Glu Glu Asp Leu Asn Gly Ala Ala
145                 150

<210>  8

<211>  462

<212>  DNA

<213>  Artificial

<220>

<223>   VHH with linker

<220>

<221>  CDS

<222>  (1)..(450)

<400> 8
acc atg gcc cag gtg aaa ctg cag cag tct ggg gga gga ttg gtg cag 48
Thr Met Ala Gln Val Lys Leu Gln Gln Ser Gly Gly Gly Leu Val Gln
1               5                   10                  15

gct ggg ggc cct ctg agg ctc tcc tgt gca gcc tct gga cgc acc ttc 96
Ala Gly Gly Pro Leu Arg Leu Ser Cys Ala Ala Ser Gly Arg Thr Phe
            20                  25                  30

agt aac tat gcc gtg ggc tgg ttc cgc cag gct cca ggg aag gag cgt 144
Ser Asn Tyr Ala Val Gly Trp Phe Arg Gln Ala Pro Gly Lys Glu Arg
        35                  40                  45

gag ttt gtc gct gct att agc cgt gat ggt ggg cgc aca tac tat gcg 192
Glu Phe Val Ala Ala Ile Ser Arg Asp Gly Gly Arg Thr Tyr Tyr Ala
    50                  55                  60

gac tcc gtg aag ggc cga ttc gcc gtc tcc aga gac tac gcc gag aac 240
Asp Ser Val Lys Gly Arg Phe Ala Val Ser Arg Asp Tyr Ala Glu Asn
65                  70                  75                  80

acg gtg tat ctg caa atg aac agc ctg aaa cct gag gac acg gcc gtt 288
Thr Val Tyr Leu Gln Met Asn Ser Leu Lys Pro Glu Asp Thr Ala Val
                85                  90                  95

tat tac tgt aac aca agg gcc tac tgg ggc cag ggg acc cag gtc acc 336
Tyr Tyr Cys Asn Thr Arg Ala Tyr Trp Gly Gln Gly Thr Gln Val Thr
            100                 105                 110

gtc tcc tca gcg cac cac agc gaa gac ccc agc tcc gcg gcc gcc cat 384
Val Ser Ser Ala His His Ser Glu Asp Pro Ser Ser Ala Ala Ala His
        115                 120                 125

cac cat cac cat cac ggg gcc gca gaa caa aaa ctc atc tca gaa gag 432
His His His His His Gly Ala Ala Glu Gln Lys Leu Ile Ser Glu Glu
    130                 135                 140

gat ctg aat ggg gcc gca tagtaacaat tg 462
Asp Leu Asn Gly Ala Ala
145                 150

<210> 9

<211> 150

<212> PRT

<213> Artificial

<400> 9

Thr Met Ala Gln Val Lys Leu Gln Gln Ser Gly Gly Gly Leu Val Gln
1               5                   10                  15

Ala Gly Gly Pro Leu Arg Leu Ser Cys Ala Ala Ser Gly Arg Thr Phe
            20                  25                  30

Ser Asn Tyr Ala Val Gly Trp Phe Arg Gln Ala Pro Gly Lys Glu Arg
        35                  40                  45

Glu Phe Val Ala Ala Ile Ser Arg Asp Gly Gly Arg Thr Tyr Tyr Ala
    50                  55                  60

Asp Ser Val Lys Gly Arg Phe Ala Val Ser Arg Asp Tyr Ala Glu Asn
65                  70                  75                  80

Thr Val Tyr Leu Gln Met Asn Ser Leu Lys Pro Glu Asp Thr Ala Val
                85                  90                  95

Tyr Tyr Cys Asn Thr Arg Ala Tyr Trp Gly Gln Gly Thr Gln Val Thr
            100                 105                 110

Val Ser Ser Ala His His Ser Glu Asp Pro Ser Ser Ala Ala Ala His
        115                 120                 125

His His His His His Gly Ala Ala Glu Gln Lys Leu Ile Ser Glu Glu
    130                 135                 140

Asp Leu Asn Gly Ala Ala
145                 150

<210>  10

<211>  471

<212>  DNA

<213>  Artificial

<220>

<223>  VHH with linker

<220>

<221>  CDS

<222>  (1)..(459)

<400>  10
acc atg gcc cag gtg aaa ctg cag cag tct ggg gga gga ttg gtg cag        48
Thr Met Ala Gln Val Lys Leu Gln Gln Ser Gly Gly Gly Leu Val Gln
1               5                   10                  15

gct ggg ggc cct ctg agg ctc tcc tgt gca gcc tct gga cgc acc ttc        96
Ala Gly Gly Pro Leu Arg Leu Ser Cys Ala Ala Ser Gly Arg Thr Phe
            20                  25                  30

agt aac tat gcc gtg ggc tgg ttc cgc cag gct cca ggg aag gag cgt       144
Ser Asn Tyr Ala Val Gly Trp Phe Arg Gln Ala Pro Gly Lys Glu Arg
        35                  40                  45

gag ttt gtc gct gct att agc cgt gat ggt ggg cgc aca tac tat gcg       192
Glu Phe Val Ala Ala Ile Ser Arg Asp Gly Gly Arg Thr Tyr Tyr Ala
    50                  55                  60

gac tcc gtg aag ggc cga ttc gcc gtc tcc aga gac tac gcc gag aac       240

Asp Ser Val Lys Gly Arg Phe Ala Val Ser Arg Asp Tyr Ala Glu Asn
65                  70                  75                  80

acg gtg tat ctg caa atg aac agc ctg aaa cct gag gac acg gcc gtt       288
Thr Val Tyr Leu Gln Met Asn Ser Leu Lys Pro Glu Asp Thr Ala Val
                85                  90                  95

tat tac tgt aac aca agg gcc tac tgg ggc cag ggg acc cag gtc acc       336
Tyr Tyr Cys Asn Thr Arg Ala Tyr Trp Gly Gln Gly Thr Gln Val Thr
            100                 105                 110

gtc tcc tca gcg cac cac agc gaa gac ccc agc tcc gcg gcc gcc cat       384
Val Ser Ser Ala His His Ser Glu Asp Pro Ser Ser Ala Ala Ala His
        115                 120                 125

cac cat cac cat cac ggg gcc gca gaa caa aaa ctc atc tca gaa gag       432
His His His His His Gly Ala Ala Glu Gln Lys Leu Ile Ser Glu Glu
    130                 135                 140

gat ctg aat agt gag aaa gat gag cta tgataacaat tg                     471
Asp Leu Asn Ser Glu Lys Asp Glu Leu
145                 150


<210>  11

<211>  153

<212>  PRT

<213>  Artificial



<400>  11

Thr Met Ala Gln Val Lys Leu Gln Gln Ser Gly Gly Gly Leu Val Gln
1               5                   10                  15


Ala Gly Gly Pro Leu Arg Leu Ser Cys Ala Ala Ser Gly Arg Thr Phe
            20                  25                  30


Ser Asn Tyr Ala Val Gly Trp Phe Arg Gln Ala Pro Gly Lys Glu Arg
        35                  40                  45


Glu Phe Val Ala Ala Ile Ser Arg Asp Gly Gly Arg Thr Tyr Tyr Ala
    50                  55                  60


Asp Ser Val Lys Gly Arg Phe Ala Val Ser Arg Asp Tyr Ala Glu Asn
65                  70                  75                  80


Thr Val Tyr Leu Gln Met Asn Ser Leu Lys Pro Glu Asp Thr Ala Val
                85                  90                  95


Tyr Tyr Cys Asn Thr Arg Ala Tyr Trp Gly Gln Gly Thr Gln Val Thr
            100                 105                 110


Val Ser Ser Ala His His Ser Glu Asp Pro Ser Ser Ala Ala Ala His
        115                 120                 125

His His His His His Gly Ala Ala Glu Gln Lys Leu Ile Ser Glu Glu
    130                 135                 140

Asp Leu Asn Ser Glu Lys Asp Glu Leu
145                 150

<210>  12

<211>  38

<212>  DNA

<213>  Artificial

<220>

<223>  synthetic insert

<400>  12
agctgcgatc gcaagcttgg taccgggaat tctctaga                              38

<210>  13

<211>  42

<212>  DNA

<213>  Artificial

<220>

<223>  synthetic insert

<400>  13
aatttctaga gaattcccgg taccaagctt gcttgcgatc gc                         42

<210>  14

<211>  31

<212>  DNA

<213>  Artificial

<220>

<223>  synthetic insert

<400>  14
tcgacccatg gcccgctagc caattggagc t                                     31

<210>  15

<211>  23

<212>  DNA

<213>  Artificial

<220>

<223>  synthetic insert

<400>  15
ccaattggct agcgggccat ggg                                              23

<210>  16

<211>  22

<212>  DNA

<213>  Artificial

<220>

<223>  primer

<400>  16
ccacccacga gggaacatcg tg                                               22

<210>  17

<211>  39

<212>  DNA

<213>  Artificial

<220>

<223>  primer

<400>  17
gaattcccat ggtttacact cgaggtcctc tccaaatga                             39

<210>  18

<211>  189

<212>  DNA

<213>  Artificial

<220>

<223>  PCR product

<400>  18
ccacccacga gggaacatcg tggaaaaaga agacgttcca accacgtctt caaagcaagt      60

ggattgatgt gatatctcca ctgacgtaag ggatgacgca caatcccact atccttcgca     120

agacccttcc tttatataag gaagttcatt tcatttggag aggacctcga gtgtaaacca     180

tgggaattc                                                              189

<210>  19

<211>  21

<212>  DNA

<213>  Artificial

<220>

<223>  sequencing primer

<400>  19
ccggcaacag gattcaatct t                                                21

<210>  20

<211>  40

<212>  DNA

<213>  Artificial

<220>

<223>  synthetic insert

<400>  20
agctccatgg gatttgttct cttttcacaa ttgccttcat                            40

<210>  21

<211>  35

<212>  DNA

<213>  Artificial

<220>

<223>  synthetic insert

<400>  21
ttcttcttgt ctctacactt ctcttattcc tagta                                 35

<210>  22

<211>  35

<212>  DNA

<213>  Artificial

<220>

<223>  synthetic insert

<400>  22
atatcccact cttgccgtgc ccaggtgcag ctgca                                35

<210>  23

<211>  48

<212>  DNA

<213>  Artificial

<220>

<223>  synthetic insert

<400>  23
gctgcacctg ggcacggcaa gagtgggata ttactaggaa taagagaa                  48

<210>  24

<211>  54

<212>  DNA

<213>  Artificial

<220>

<223>  synthetic insert

<400>  24
gtgtagagac aagaagaaat gaaggcaatt gtgaaaagag aacaaatccc atgg           54

<210>  25

<211>  24

<212>  DNA

<213>  Artificial

<220>

<223>  synthetic insert

<400>  25
ggaaacagct atgaccatga ttac                                            24

<210>  26

<211>  20

<212>  DNA

<213>  Artificial

<220>

<223>  synthetic insert

<400>  26
tttcccagtc acgacgttgt                                                    20

<210>  27

<211>  107

<212>  DNA

<213>  Artificial

<220>

<223>  synthetic fragment encoding PRIa leader

<220>

<221>  CDS

<222>  (3)..(104)

<400>  27
cc atg gga ttt gtt ctc ttt tca caa ttg cct tca ttt ctt ctt gtc        47
   Met Gly Phe Val Leu Phe Ser Gln Leu Pro Ser Phe Leu Leu Val
   1               5                   10                  15

tct aca ctt ctc tta ttc cta gta ata tcc cac tct tgc cgt gcc cag       95
Ser Thr Leu Leu Leu Phe Leu Val Ile Ser His Ser Cys Arg Ala Gln
                20                  25                  30

gtg cag ctg cag                                                      107
Val Gln Leu

<210>  28

<211>  34

<212>  PRT

<213>  Artificial

<400>  28

Met Gly Phe Val Leu Phe Ser Gln Leu Pro Ser Phe Leu Leu Val Ser
1               5                   10                  15

Thr Leu Leu Leu Phe Leu Val Ile Ser His Ser Cys Arg Ala Gln Val
            20                  25                  30

Gln Leu

<210> 29

<211> 21

<212> DNA

<213> Artificial

<220>

<223> PCR primer

<400> 29
gacatcccat ggcaagcatc a 21

<210> 30

<211> 21

<212> DNA

<213> Artificial

<220>

<223> PCR primer

<400> 30
aagcttgtta acagccctta a 21

<210> 31

<211> 21

<212> DNA

<213> Artificial

<220>

<223> PCR primer

<400> 31
agggctgtta acaaacttga t 21

<210> 32

<211> 44

<212> DNA

<213> Artificial

<220>

<223>  PCR primer

<400>  32
agactgctgc agctgcacct gctttccaca aacaatggta gctg                        44


<210>  33

<211>  19

<212>  DNA

<213>  Artificial



<220>

<223>  sequencing primer

<400>  33
aattaaccct cactaaagg                                                    19


<210>  34

<211>  254

<212>  DNA

<213>  Artificial



<220>

<223>   GBSS leader

<220>

<221>  CDS

<222>  (3)..(254)



<400>  34
cc atg gca agc atc aca gct tca cac cac ttt gtg tca aga agc caa          47
   Met Ala Ser Ile Thr Ala Ser His His Phe Val Ser Arg Ser Gln
   1               5                   10                  15

act tca cta gac acc aaa tca acc ttg tca cag ata gga ctc agg aac         95
Thr Ser Leu Asp Thr Lys Ser Thr Leu Ser Gln Ile Gly Leu Arg Asn
                20                  25                  30

cat act ctg act cac aat ggt tta agg gct gtt aac aaa ctt gat ggg        143
His Thr Leu Thr His Asn Gly Leu Arg Ala Val Asn Lys Leu Asp Gly
            35                  40                  45

ctc caa tca aga act aat act aag gta aca ccc aag atg gca tcc aga        191
Leu Gln Ser Arg Thr Asn Thr Lys Val Thr Pro Lys Met Ala Ser Arg
        50                  55                  60

act gag acc aag aga cct gga tgc tca gct acc att gtt tgt gga aaa        239
Thr Glu Thr Lys Arg Pro Gly Cys Ser Ala Thr Ile Val Cys Gly Lys
    65                  70                  75

cag gtg cag ctg cag                                                    254
Gln Val Gln Leu Gln
80

<210>  35

<211>  84

<212>  PRT

<213>  Artificial

<400>  35

Met Ala Ser Ile Thr Ala Ser His His Phe Val Ser Arg Ser Gln Thr
1               5                   10                  15

Ser Leu Asp Thr Lys Ser Thr Leu Ser Gln Ile Gly Leu Arg Asn His
            20                  25                  30

Thr Leu Thr His Asn Gly Leu Arg Ala Val Asn Lys Leu Asp Gly Leu
        35                  40                  45

Gln Ser Arg Thr Asn Thr Lys Val Thr Pro Lys Met Ala Ser Arg Thr
    50                  55                  60

Glu Thr Lys Arg Pro Gly Cys Ser Ala Thr Ile Val Cys Gly Lys Gln
65                  70                  75                  80

Val Gln Leu Gln

<210>  36

<211>  18

<212>  DNA

<213>  Artificial

<220>

<223>  synthetic insert

<400>  36
catgcaggtg cagctgca                                                    18

<210>  37

<211>  10

<212>  DNA

<213>  Artificial

<220>

<223>  synthetic insert

<400>  37
gctgcacctg                                                              10


<210>  38

<211>  24

<212>  DNA

<213>  Artificial



<220>

<223>  PCR primer

<400>  38
cgcaagaccc ttcctctata taag                                              24


<210>  39

<211>  60

<212>  DNA

<213>  Artificial



<220>

<223>  PCR primer

<400>  39
gagctcgaat tcttattata gctcatcttt ctctgaattc agatcctctt ctgagatgag      60


<210>  40

<211>  26

<212>  PRT

<213>  Artificial



<220>

<223>  linker

<400>  40

Thr Val Thr Val Ser Ser Gly Gly Gly Gly Ser Gly Gly Gly Gly Ser
1               5                   10                  15

Gly Gly Gly Gly Ser Asp Ile Glu Leu Thr
            20                  25

<210>  41

<211>  20

<212>  DNA

<213>  Artificial

<220>

<223>  PCR primer

<400>  41
atcctcaact tccaatcaga                                                    20

<210>  42

<211>  19

<212>  DNA

<213>  Artificial

<220>

<223>  PCR primer

<400>  42
ttcttgagag atagcttga                                                     19

<210>  43

<211>  30

<212>  DNA

<213>  Artificial

<220>

<223>  synthetic insert

<400>  43
gatcccatgg cccgctagcc aattggagct                                         30

<210>  44

<211>  22

<212>  DNA

<213>  Artificial

<220>

<223>  synthetic insert

<400> 44
ccaattggct agcgggccat gg 22

<210> 45

<211> 29

<212> DNA

<213> Artificial

<220>

<223> synthetic insert

<400> 45
gatccacctc gagtgtaaac catggcccg 29

<210> 46

<211> 29

<212> DNA

<213> Artificial

<220>

<223> synthetic insert

<400> 46
ctagcgggcc atggtttaca ctcgaggtg 29

<210> 47

<211> 20

<212> DNA

<213> Artificial

<220>

<223> PCR primer

<400> 47
attgcctacg gcagccgctg 20

<210> 48

<211> 51

<212> DNA

<213> Artificial

<220>

<223>  PCR primer

<400>  48
tccaaccaat tgttactatg cggccccatt cagatcctct tctgagatga g          51


<210>  49

<211>  25

<212>  DNA

<213>  Artificial



<220>

<223>  sequencing primer

<400>  49
gtctgtctaa agtaaagtag atgcg                                       25


<210>  50

<211>  60

<212>  DNA

<213>  Artificial



<220>

<223>  PCR primer

<400>  50
tccaaccaat tgttatcata gctcatcttt ctcactattc agatcctctt ctgagatgag    60


<210>  51

<211>  29

<212>  DNA

<213>  Artificial



<220>

<223>  PCR primer

<400>  51
agtcccccat ggtacgtcct gtagaaacc                                   29


<210>  52

<211>  25

<212>  DNA

<213>  Artificial

<220>

<223>  PCR primer

<400>  52
cgttttcgtc ggtaatcacc attcc                                          25

<210>  53

<211>  24

<212>  DNA

<213>  Artificial

<220>

<223>  sequencing primer

<400>  53
cgcaagaccc ttcctttata taag                                           24

<210>  54

<211>  1154

<212>  DNA

<213>  Artificial

<220>

<223>  HCV33-hinge-CH2-CH3

<220>

<221>  CDS

<222>  (3)..(1136)

<400>  54
cc atg gag gtg cag ctg cag gag tca ggg gga gga ttg gtg cag gct       47
   Met Glu Val Gln Leu Gln Glu Ser Gly Gly Gly Leu Val Gln Ala
   1               5                   10                  15

ggg ggc tct ctg aga ctc tcc tgt gca gcc tcg gga cgc gcc acc agt      95
Gly Gly Ser Leu Arg Leu Ser Cys Ala Ala Ser Gly Arg Ala Thr Ser
                20                  25                  30

ggt cat ggt cac tat ggt atg ggc tgg ttc cgc cag gtt cca ggg aag     143
Gly His Gly His Tyr Gly Met Gly Trp Phe Arg Gln Val Pro Gly Lys
            35                  40                  45

gag cgt gag ttt gtc gca gct att agg tgg agt ggt aaa gag aca tgg     191
Glu Arg Glu Phe Val Ala Ala Ile Arg Trp Ser Gly Lys Glu Thr Trp
        50                  55                  60

tat aaa gac tcc gtg aag ggc cga ttc acc atc tcc aga gat aac gcc     239
Tyr Lys Asp Ser Val Lys Gly Arg Phe Thr Ile Ser Arg Asp Asn Ala
    65                  70                  75

aag act acg gtt tat ctg caa atg aac agc ctg aaa cct gaa gat acg     287
Lys Thr Thr Val Tyr Leu Gln Met Asn Ser Leu Lys Pro Glu Asp Thr
80                  85                  90                  95

gcc gtt tat tat tgt gcc gct cga ccg gtc cgc gtg gat gat att tcc     335
Ala Val Tyr Tyr Cys Ala Ala Arg Pro Val Arg Val Asp Asp Ile Ser
                100                 105                 110

ctg ccg gtt ggg ttt gac tac tgg ggc cag ggg acc cag gtc acc gtc     383
Leu Pro Val Gly Phe Asp Tyr Trp Gly Gln Gly Thr Gln Val Thr Val
            115                 120                 125

tcc tca gaa ccc aag aca cca aaa cca caa cca caa cca caa cca caa     431
Ser Ser Glu Pro Lys Thr Pro Lys Pro Gln Pro Gln Pro Gln Pro Gln
        130                 135                 140

cca caa ccc aat cct aca aca gaa tcc aag tgt ccc aaa tgt cca gcc     479
Pro Gln Pro Asn Pro Thr Thr Glu Ser Lys Cys Pro Lys Cys Pro Ala
    145                 150                 155

cct gag ctc ctg gga ggg ccc tca gtc ttc atc ttc ccc ccg aaa ccc     527
Pro Glu Leu Leu Gly Gly Pro Ser Val Phe Ile Phe Pro Pro Lys Pro
160                 165                 170                 175

aag gac gtc ctc tcc att tct ggg agg ccc gag gtc acg tgc gtt gtg     575
Lys Asp Val Leu Ser Ile Ser Gly Arg Pro Glu Val Thr Cys Val Val
                180                 185                 190

gta gac gtg ggc cag gaa gac ccc gag gtc agt ttc aac tgg tac att     623
Val Asp Val Gly Gln Glu Asp Pro Glu Val Ser Phe Asn Trp Tyr Ile
            195                 200                 205

gat ggc gca gag gtg cga acg gcc aac acg agg cca aaa gag gaa cag     671
Asp Gly Ala Glu Val Arg Thr Ala Asn Thr Arg Pro Lys Glu Glu Gln
        210                 215                 220

ttc aac agc acg tac cgc gtg gtc agc gtc ctg ccc atc cag cac cag     719
Phe Asn Ser Thr Tyr Arg Val Val Ser Val Leu Pro Ile Gln His Gln
    225                 230                 235

gac tgg ctg acg ggg aaa gag ttc aaa tgc aag gtc aac aac aaa gct     767
Asp Trp Leu Thr Gly Lys Glu Phe Lys Cys Lys Val Asn Asn Lys Ala
240                 245                 250                 255

ctc ccg gcc ccc atc gag aag acc atc tcc aag gcc aaa ggg cag acc     815
Leu Pro Ala Pro Ile Glu Lys Thr Ile Ser Lys Ala Lys Gly Gln Thr
                260                 265                 270

cgg gag ccg cag gtg tac gcc ctg gcc cca cac cgg gaa gag ctg gcc     863
Arg Glu Pro Gln Val Tyr Ala Leu Ala Pro His Arg Glu Glu Leu Ala
            275                 280                 285

aag gac acc gtg agc gta acc tgc ctg gtc aaa ggc ttc tac cca cct     911
Lys Asp Thr Val Ser Val Thr Cys Leu Val Lys Gly Phe Tyr Pro Pro
        290                 295                 300

gat atc aac gtt gag tgg cag agg aac ggt cag ccg gag tca gag ggc     959
Asp Ile Asn Val Glu Trp Gln Arg Asn Gly Gln Pro Glu Ser Glu Gly
    305                 310                 315

acc tac gcc acc acg cca ccc cag ctg gac aac gac ggg acc tac ttc     1007
Thr Tyr Ala Thr Thr Pro Pro Gln Leu Asp Asn Asp Gly Thr Tyr Phe
320                 325                 330                 335

ctc tac agc aag ctc tcg gtg gga aag aac acg tgg cag cgg gga gaa      1055
Leu Tyr Ser Lys Leu Ser Val Gly Lys Asn Thr Trp Gln Arg Gly Glu
                340                 345                 350

acc ttc acc tgt gtg gtg atg cac gag gcc ctg cac aac cac tac acc      1103
Thr Phe Thr Cys Val Val Met His Glu Ala Leu His Asn His Tyr Thr
            355                 360                 365

cag aaa tcc atc acc cag tct tcg ggt aaa taa taagaattcg agctcgaa      1154
Gln Lys Ser Ile Thr Gln Ser Ser Gly Lys
        370                 375

<210>  55

<211>  377

<212>  PRT

<213>  Artificial

<400>  55

Met Glu Val Gln Leu Gln Glu Ser Gly Gly Gly Leu Val Gln Ala Gly
1               5                   10                  15

Gly Ser Leu Arg Leu Ser Cys Ala Ala Ser Gly Arg Ala Thr Ser Gly
            20                  25                  30

His Gly His Tyr Gly Met Gly Trp Phe Arg Gln Val Pro Gly Lys Glu
        35                  40                  45

Arg Glu Phe Val Ala Ala Ile Arg Trp Ser Gly Lys Glu Thr Trp Tyr
    50                  55                  60

Lys Asp Ser Val Lys Gly Arg Phe Thr Ile Ser Arg Asp Asn Ala Lys
65                  70                  75                  80

Thr Thr Val Tyr Leu Gln Met Asn Ser Leu Lys Pro Glu Asp Thr Ala
                85                  90                  95

Val Tyr Tyr Cys Ala Ala Arg Pro Val Arg Val Asp Asp Ile Ser Leu
            100                 105                 110

Pro Val Gly Phe Asp Tyr Trp Gly Gln Gly Thr Gln Val Thr Val Ser
        115                 120                 125

Ser Glu Pro Lys Thr Pro Lys Pro Gln Pro Gln Pro Gln Pro Gln Pro
    130                 135                 140

Gln Pro Asn Pro Thr Thr Glu Ser Lys Cys Pro Lys Cys Pro Ala Pro
145                 150                 155                 160

Glu Leu Leu Gly Gly Pro Ser Val Phe Ile Phe Pro Pro Lys Pro Lys
                165                 170                 175

Asp Val Leu Ser Ile Ser Gly Arg Pro Glu Val Thr Cys Val Val Val
            180                 185                 190

Asp Val Gly Gln Glu Asp Pro Glu Val Ser Phe Asn Trp Tyr Ile Asp
        195                 200                 205

Gly Ala Glu Val Arg Thr Ala Asn Thr Arg Pro Lys Glu Glu Gln Phe
    210                 215                 220

Asn Ser Thr Tyr Arg Val Val Ser Val Leu Pro Ile Gln His Gln Asp
225                 230                 235                 240

Trp Leu Thr Gly Lys Glu Phe Lys Cys Lys Val Asn Asn Lys Ala Leu
                245                 250                 255

Pro Ala Pro Ile Glu Lys Thr Ile Ser Lys Ala Lys Gly Gln Thr Arg
            260                 265                 270

Glu Pro Gln Val Tyr Ala Leu Ala Pro His Arg Glu Glu Leu Ala Lys
        275                 280                 285

Asp Thr Val Ser Val Thr Cys Leu Val Lys Gly Phe Tyr Pro Pro Asp
    290                 295                 300

Ile Asn Val Glu Trp Gln Arg Asn Gly Gln Pro Glu Ser Glu Gly Thr
305                 310                 315                 320

Tyr Ala Thr Thr Pro Pro Gln Leu Asp Asn Asp Gly Thr Tyr Phe Leu
                325                 330                 335

Tyr Ser Lys Leu Ser Val Gly Lys Asn Thr Trp Gln Arg Gly Glu Thr
            340                 345                 350

Phe Thr Cys Val Val Met His Glu Ala Leu His Asn His Tyr Thr Gln
        355                 360                 365

Lys Ser Ile Thr Gln Ser Ser Gly Lys
    370                 375

<210>  56

<211>  1172

<212>  DNA

<213>  Artificial

<220>

<223>  hinge-HCV33-CH2-CH3-SEKDEL

<220>

<221>  CDS

<222>  (3)..(1154)

<400>  56
cc atg gag gtg cag ctg cag gag tca ggg gga gga ttg gtg cag gct        47
   Met Glu Val Gln Leu Gln Glu Ser Gly Gly Gly Leu Val Gln Ala
   1               5                   10                  15

ggg ggc tct ctg aga ctc tcc tgt gca gcc tcg gga cgc gcc acc agt        95
Gly Gly Ser Leu Arg Leu Ser Cys Ala Ala Ser Gly Arg Ala Thr Ser
                20                  25                  30

ggt cat ggt cac tat ggt atg ggc tgg ttc cgc cag gtt cca ggg aag       143
Gly His Gly His Tyr Gly Met Gly Trp Phe Arg Gln Val Pro Gly Lys
            35                  40                  45

gag cgt gag ttt gtc gca gct att agg tgg agt ggt aaa gag aca tgg       191
Glu Arg Glu Phe Val Ala Ala Ile Arg Trp Ser Gly Lys Glu Thr Trp
        50                  55                  60

tat aaa gac tcc gtg aag ggc cga ttc acc atc tcc aga gat aac gcc       239
Tyr Lys Asp Ser Val Lys Gly Arg Phe Thr Ile Ser Arg Asp Asn Ala
    65                  70                  75

aag act acg gtt tat ctg caa atg aac agc ctg aaa cct gaa gat acg       287
Lys Thr Thr Val Tyr Leu Gln Met Asn Ser Leu Lys Pro Glu Asp Thr
80                  85                  90                  95

gcc gtt tat tat tgt gcc gct cga ccg gtc cgc gtg gat gat att tcc       335
Ala Val Tyr Tyr Cys Ala Ala Arg Pro Val Arg Val Asp Asp Ile Ser
                100                 105                 110

ctg ccg gtt ggg ttt gac tac tgg ggc cag ggg acc cag gtc acc gtc       383
Leu Pro Val Gly Phe Asp Tyr Trp Gly Gln Gly Thr Gln Val Thr Val
            115                 120                 125

tcc tca gaa ccc aag aca cca aaa cca caa cca caa cca caa cca caa       431
Ser Ser Glu Pro Lys Thr Pro Lys Pro Gln Pro Gln Pro Gln Pro Gln
        130                 135                 140

cca caa ccc aat cct aca aca gaa tcc aag tgt ccc aaa tgt cca gcc       479
Pro Gln Pro Asn Pro Thr Thr Glu Ser Lys Cys Pro Lys Cys Pro Ala
    145                 150                 155

cct gag ctc ctg gga ggg ccc tca gtc ttc atc ttc ccc ccg aaa ccc       527
Pro Glu Leu Leu Gly Gly Pro Ser Val Phe Ile Phe Pro Pro Lys Pro
160                 165                 170                 175

aag gac gtc ctc tcc att tct ggg agg ccc gag gtc acg tgc gtt gtg       575
Lys Asp Val Leu Ser Ile Ser Gly Arg Pro Glu Val Thr Cys Val Val
                180                 185                 190

gta gac gtg ggc cag gaa gac ccc gag gtc agt ttc aac tgg tac att       623
Val Asp Val Gly Gln Glu Asp Pro Glu Val Ser Phe Asn Trp Tyr Ile
            195                 200                 205

gat ggc gca gag gtg cga acg gcc aac acg agg cca aaa gag gaa cag       671
Asp Gly Ala Glu Val Arg Thr Ala Asn Thr Arg Pro Lys Glu Glu Gln
        210                 215                 220

ttc aac agc acg tac cgc gtg gtc agc gtc ctg ccc atc cag cac cag       719
Phe Asn Ser Thr Tyr Arg Val Val Ser Val Leu Pro Ile Gln His Gln
    225                 230                 235

gac tgg ctg acg ggg aaa gag ttc aaa tgc aag gtc aac aac aaa gct        767
Asp Trp Leu Thr Gly Lys Glu Phe Lys Cys Lys Val Asn Asn Lys Ala
240                 245                 250                 255

ctc ccg gcc ccc atc gag aag acc atc tcc aag gcc aaa ggg cag acc        815
Leu Pro Ala Pro Ile Glu Lys Thr Ile Ser Lys Ala Lys Gly Gln Thr
                260                 265                 270

cgg gag ccg cag gtg tac gcc ctg gcc cca cac cgg gaa gag ctg gcc        863
Arg Glu Pro Gln Val Tyr Ala Leu Ala Pro His Arg Glu Glu Leu Ala
            275                 280                 285

aag gac acc gtg agc gta acc tgc ctg gtc aaa ggc ttc tac cca cct        911
Lys Asp Thr Val Ser Val Thr Cys Leu Val Lys Gly Phe Tyr Pro Pro
        290                 295                 300

gat atc aac gtt gag tgg cag agg aac ggt cag ccg gag tca gag ggc        959
Asp Ile Asn Val Glu Trp Gln Arg Asn Gly Gln Pro Glu Ser Glu Gly
    305                 310                 315

acc tac gcc acc acg cca ccc cag ctg gac aac gac ggg acc tac ttc        1007
Thr Tyr Ala Thr Thr Pro Pro Gln Leu Asp Asn Asp Gly Thr Tyr Phe
320                 325                 330                 335

ctc tac agc aag ctc tcg gtg gga aag aac acg tgg cag cgg gga gaa        1055
Leu Tyr Ser Lys Leu Ser Val Gly Lys Asn Thr Trp Gln Arg Gly Glu
                340                 345                 350

acc ttc acc tgt gtg gtg atg cac gag gcc ctg cac aac cac tac acc        1103
Thr Phe Thr Cys Val Val Met His Glu Ala Leu His Asn His Tyr Thr
            355                 360                 365

cag aaa tcc atc acc cag tct tcg ggt aaa tct gag aaa gat gag cta        1151
Gln Lys Ser Ile Thr Gln Ser Ser Gly Lys Ser Glu Lys Asp Glu Leu
        370                 375                 380

taa taagaattcg agctcgaa                                                1172

<210>  57

<211>  383

<212>  PRT

<213>  Artificial

<400>  57

Met Glu Val Gln Leu Gln Glu Ser Gly Gly Gly Leu Val Gln Ala Gly
1               5                   10                  15

Gly Ser Leu Arg Leu Ser Cys Ala Ala Ser Gly Arg Ala Thr Ser Gly
            20                  25                  30

His Gly His Tyr Gly Met Gly Trp Phe Arg Gln Val Pro Gly Lys Glu
        35                  40                  45

Arg Glu Phe Val Ala Ala Ile Arg Trp Ser Gly Lys Glu Thr Trp Tyr
    50                  55                  60

Lys Asp Ser Val Lys Gly Arg Phe Thr Ile Ser Arg Asp Asn Ala Lys
65                  70                  75                  80

Thr Thr Val Tyr Leu Gln Met Asn Ser Leu Lys Pro Glu Asp Thr Ala
                85                  90                  95

Val Tyr Tyr Cys Ala Ala Arg Pro Val Arg Val Asp Asp Ile Ser Leu
            100                 105                 110

Pro Val Gly Phe Asp Tyr Trp Gly Gln Gly Thr Gln Val Thr Val Ser
        115                 120                 125

Ser Glu Pro Lys Thr Pro Lys Pro Gln Pro Gln Pro Gln Pro Gln Pro
    130                 135                 140

Gln Pro Asn Pro Thr Thr Glu Ser Lys Cys Pro Lys Cys Pro Ala Pro
145                 150                 155                 160

Glu Leu Leu Gly Gly Pro Ser Val Phe Ile Phe Pro Pro Lys Pro Lys
                165                 170                 175

Asp Val Leu Ser Ile Ser Gly Arg Pro Glu Val Thr Cys Val Val Val
            180                 185                 190

Asp Val Gly Gln Glu Asp Pro Glu Val Ser Phe Asn Trp Tyr Ile Asp
        195                 200                 205

Gly Ala Glu Val Arg Thr Ala Asn Thr Arg Pro Lys Glu Glu Gln Phe
    210                 215                 220

Asn Ser Thr Tyr Arg Val Val Ser Val Leu Pro Ile Gln His Gln Asp
225                 230                 235                 240

Trp Leu Thr Gly Lys Glu Phe Lys Cys Lys Val Asn Asn Lys Ala Leu
                245                 250                 255

Pro Ala Pro Ile Glu Lys Thr Ile Ser Lys Ala Lys Gly Gln Thr Arg
            260                 265                 270

Glu Pro Gln Val Tyr Ala Leu Ala Pro His Arg Glu Glu Leu Ala Lys
        275                 280                 285

Asp Thr Val Ser Val Thr Cys Leu Val Lys Gly Phe Tyr Pro Pro Asp
    290                 295                 300

Ile Asn Val Glu Trp Gln Arg Asn Gly Gln Pro Glu Ser Glu Gly Thr
305                 310                 315                 320

Tyr Ala Thr Thr Pro Pro Gln Leu Asp Asn Asp Gly Thr Tyr Phe Leu
                325                 330                 335

Tyr Ser Lys Leu Ser Val Gly Lys Asn Thr Trp Gln Arg Gly Glu Thr
            340                 345                 350

Phe Thr Cys Val Val Met His Glu Ala Leu His Asn His Tyr Thr Gln
        355                 360                 365

Lys Ser Ile Thr Gln Ser Ser Gly Lys Ser Glu Lys Asp Glu Leu
    370                 375                 380

<210>  58

<211>  31

<212>  DNA

<213>  Artificial

<220>

<223>  PCR primer

<400>  58
aggggaccca ggtcaccgtc tcctcagaac c                                    31

<210>  59

<211>  37

<212>  DNA

<213>  Artificial

<220>

<223>  PCR primer

<400>  59
gagctttgtt gttgaccttg catttgaact ctttccc                              37

<210>  60

<211>  26

<212>  DNA

<213>  Artificial

<220>

<223>  PCR primer

<400>  60
caaatgcaag gtcaacaaca aagctc                                          26

<210>  61

<211>  42

<212>  DNA

<213>  Artificial

<220>

<223>  PCR primer

<400>  61
ttcgagctcg aattcttatt atttacccga agactgggtg at                          42

<210>  62

<211>  27

<212>  DNA

<213>  Artificial

<220>

<223>  sequencing primer

<400>  62
ctgaggagac ggtgacctgg gtcccct                                           27

<210>  63

<211>  46

<212>  DNA

<213>  Artificial

<220>

<223>  PCR primer

<400>  63
agcccctgag ctcctgggag ggccctcagt cttcatcttc cccccg                      46

<210>  64

<211>  61

<212>  DNA

<213>  Artificial

<220>

<223>  PCR primer

<400>  64
ttcgagctcg aattcttatt atttacccga agactgggtg atggatttct gggtgtagtg      60

g                                                                    61

<210>  65

<211>  79

<212>  DNA

<213>  Artificial

<220>

<223>  PCR primer

<400>  65
ttcgagctcg aattcttatt atagctcatc tttctcagat ttacccgaag actgggtgat      60

ggatttctgg gtgtagtgg                                                   79

<210>  66

<211>  461

<212>  DNA

<213>  Artificial

<220>

<223>  VHH with linker

<220>

<221>  CDS

<222>  (3)..(449)

<400>  66
cc atg gcc cag gtg cag ctg cag gag tct ggg gga ggc ttg gtg cag        47
   Met Ala Gln Val Gln Leu Gln Glu Ser Gly Gly Gly Leu Val Gln
   1               5                   10                  15

gct ggg ggg tct ctg agg ctc tcc tgt gca gcc tct gga agc att ttc       95
Ala Gly Gly Ser Leu Arg Leu Ser Cys Ala Ala Ser Gly Ser Ile Phe
                20                  25                  30

aga cgt ccg cat atg ggt tgg ttc cgc cag gct cca ggg cag gag cgc      143
Arg Arg Pro His Met Gly Trp Phe Arg Gln Ala Pro Gly Gln Glu Arg
            35                  40                  45

gag ttg gtc gca ctg att tct gcg ggt ggt cgt aca tgg tat gca gac      191
Glu Leu Val Ala Leu Ile Ser Ala Gly Gly Arg Thr Trp Tyr Ala Asp
        50                  55                  60

tcc gtg aag ggc cga ttc acc atc tcc aga gac aac gcc aag aac acg      239
Ser Val Lys Gly Arg Phe Thr Ile Ser Arg Asp Asn Ala Lys Asn Thr
    65                  70                  75

ctg tat ctg caa atg aac agc ctg aaa cct gag gac acg gcc gtt tat      287

Leu Tyr Leu Gln Met Asn Ser Leu Lys Pro Glu Asp Thr Ala Val Tyr
80                  85                  90                  95

tac tgt act gcc ggg ggt tcg tac tgg ggc cag ggg acc cag gtc acc       335
Tyr Cys Thr Ala Gly Gly Ser Tyr Trp Gly Gln Gly Thr Gln Val Thr
                100                 105                 110

gtc gcc tca gaa ccc aag aca cca aaa cca caa cca gcg gcc gcc cat       383
Val Ala Ser Glu Pro Lys Thr Pro Lys Pro Gln Pro Ala Ala Ala His
            115                 120                 125

cac cat cac cat cac ggg gcc gca gaa caa aaa ctc atc tca gaa gag       431
His His His His His Gly Ala Ala Glu Gln Lys Leu Ile Ser Glu Glu
    130                 135                 140

gat ctg aat ggg gcc gca tagtaacaat tg                                 461
Asp Leu Asn Gly Ala Ala
    145

<210>  67

<211>  149

<212>  PRT

<213>  Artificial

<400>  67

Met Ala Gln Val Gln Leu Gln Glu Ser Gly Gly Gly Leu Val Gln Ala
1               5                   10                  15

Gly Gly Ser Leu Arg Leu Ser Cys Ala Ala Ser Gly Ser Ile Phe Arg
            20                  25                  30

Arg Pro His Met Gly Trp Phe Arg Gln Ala Pro Gly Gln Glu Arg Glu
        35                  40                  45

Leu Val Ala Leu Ile Ser Ala Gly Gly Arg Thr Trp Tyr Ala Asp Ser
    50                  55                  60

Val Lys Gly Arg Phe Thr Ile Ser Arg Asp Asn Ala Lys Asn Thr Leu
65                  70                  75                  80

Tyr Leu Gln Met Asn Ser Leu Lys Pro Glu Asp Thr Ala Val Tyr Tyr
                85                  90                  95

Cys Thr Ala Gly Gly Ser Tyr Trp Gly Gln Gly Thr Gln Val Thr Val
            100                 105                 110

Ala Ser Glu Pro Lys Thr Pro Lys Pro Gln Pro Ala Ala Ala His His
        115                 120                 125

His His His His Gly Ala Ala Glu Gln Lys Leu Ile Ser Glu Glu Asp
    130                 135                 140

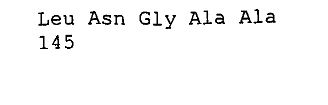

## Description

### FIELD OF THE INVENTION

The present invention is in the field of applied biotechnology and relates in particular to an economic way of producing antibodies, or more particularly fragments thereof, in plants.

### BACKGROUND OF THE INVENTION

The production of antibodies in microbial or plant systems can be advantageous for a number of applications.

Firstly microbial or plant sources can be used as bioreactors. In this situation the antibodies and/or their derivatives are produced on a large scale in modified organisms. The use of such 'bioreactors', especially plants has the advantage that scale up to produce large quantities e.g. more than 100 kg or even more than 1000 kg of antibodies is relatively easy and does not require significant investments in harvesting or processing equipment.

Alternatively antibodies and/or their derivatives can be produced in plants with the aim of reprogramming the plant metabolism or to improve defence mechanisms of said plant. The antibodies to be produced are chosen such that they target specific enzymes to modulate their activity and/or to provide the plant with protection against pathogens such as parasites and viruses.

Several attempts have been made to express antibodies or fragments thereof in micro-organisms or plants.

Evidence from the literature suggests that where it is desired to express antibodies in microbial systems, the most favourable results are obtained using smaller fragments. Whole antibodies and larger antibody fragments, such as Fab fragments, are very difficult if not impossible to produce. Smaller molecules such as single chain antibody fragments (scFv) are somewhat easier. Generally, however the best results are obtained if even smaller molecules such as heavy chain variable fragments (HCV) are produced (see, for example, WO 94/25591 (Unilever)).

In plant host systems, both large molecules, such as complete murine antibodies and smaller murine antibody fragments such as single chain Fv (scFv) fragments are capable of being expressed.

The production of functional complete murine antibodies in plants was first reported by Hiatt et al, Nature, 342, 76-78 (1989). Subsequent reports of the functional expression of murine monoclonal antibodies in plants include Düring et al, Plant Molecular Biology, 15, 281-293, (1990) and Ma et al, European Journal of Immunology, 24, 131-138, (1994).

Transgenic tobacco plants expressing a synthetic gene encoding an antigen binding single chain Fv protein (scFv) and which produce functional scFv protein have been described by Owen et al, Biotechnology, 10, 790-794, (1992). The expression of functional (that is having antigen-binding activity) scFv protein in transgenic plants has also been described in other reports in the literature, see, for example, Tavlaoraki et al, Nature, 366, 469-472 (1993).

Given that the production of functional complete antibodies requires the correct assembly, via covalent and non-covalent interactions, of both the antibody heavy and light chains, it might have been expected that expression of smaller antibody fragments, with their less stringent assembly requirements, would be advantageous. It has however been reported that generally, in practice, better yields are achieved with plants transformed with complete murine antibodies rather than small fragments (Ma et al, Science, 268, 716-719 (1995))

Furthermore, it is the experience of the present inventors that the expression of genes encoding scFv proteins in plants is not reliably reproducible and hence such a process would not readily lend itself to large scale production. Moreover, expression of genes encoding scFv molecules in plants can have an undesirable effect on plant cell morphology.

Benevenuto et al, Plant Molecular Biology, 17, 865-874 (1991) describe attempts to express smaller isolated murine heavy chain variable domain antibody fragments in plants. Successful expression is reported but there is no indication that binding affinity is retained.

For many purposes, it is important to be able to target antibodies or fragments thereof to specific cellular compartments. For example, where maximisation of yield is desired, targeting to the ER or the apoplastic space can help by providing a less reducing, more stable environment than is present in the cytoplasm. On the other hand, where it is desired to interfere with the activity of specific enzymes, in order to engineer metabolic changes in a plant, it is necessary to ensure that the antibodies are directed to the particular compartment in which the enzyme is active.

In order to direct the expression of the desired protein to a specific cellular compartment within a plant cell, the antibody proteins are fused to specific targeting sequences (peptides) where required. Expression in the chloroplast requires an N-terminal chloroplast targeting sequence (generally termed transit peptide) which is cleaved off upon entry into the chloroplast (reviewed in Keegstra & Cline (2000) Plant Cell 11, 557-570). There is little sequence homology among these transit peptides except that they are often rich in hydroxylated amino acides (eg serine), are deficient in acidic amino acids and they can vary in length from 30 to >100 amino acids. Various transit peptides fused to heterologous proteins have been used successfully to direct the protein to the chloroplast however there are no clear rules that will guarantee correct targeting and cleavage of the fusion protein. Some groups have used an exact fusion of the transit peptide to the heterologous protein; for example the transit peptide of the small subunit of rubisco from petunia has been used in this way (Fray et al (2000) Nature Biotech 17, 1017-1020). Others have used transit peptides with two amino acids of the mature protein, eg potato granule-bound starch synthase (GBSS) (Kortsee et al (1996) Plant Journal 10, 83-90), and some have used as many as 23 amino acids of the mature protein eg the small subunit of rubisco from pea (Nawrath et al (1994) Proc. Natl. Acad. Sci USA 91, 12760-12764). Another group used the transit peptide of the Arabidopsis small subunit of rubisco plus 24 amino acids of the mature protein followed by a repeat of six amino acids before the cleavage site and an additional two amino acids of the mature rubisco protein (Wong et al (1992) Plant Mol. Biol. 20, 81-93).

Proteins that enter the secretory system have N-terminal targeting peptides generally known as "signal peptides or sequences" which direct them initially to the endoplasmic reticulum (ER) whence, depending on additional sequences, the proteins are sorted either to vacuoles or are secreted from the cell. In addition, proteins which enter the ER and have a carboxy terminal peptide KDEL are retained within the ER membrane system. For location in the cytoplasm, no targeting sequence is required.

The targeting of classical conventional antibodies (ie IgG, scFv etc) to various cellular compartments of plants has been reviewed in Conrad and Fielder (1998) Plant Molecular Biology 38, 101-109. Most commonly the homologous signal sequence of the murine IgG (e.g. CEA66E3) has been used to direct expression of the antibody via the secretory pathway, although the signal sequences from plants (PR1a, legumin B4, 2S storage protein) and even bacteria (pelB) and yeast have been used successfully. Generally the highest expression levels were obtained by the use of a signal peptide and C-terminal KDEL ER retention signal. There are no reports of successful targeting of antibodies to chloroplasts using a transit peptide as the targeting sequence. However, Düring et al (1990) Plant Mol. Biol. 15, 281-293, reported that when the barley α-amylase signal peptide was fused to the light and heavy chains of a monoclonal antibody, there was some apparent aberrant localisation of antibody within the chloroplast although the majority of the labelling was in the ER. The authors had no explanation for this observation and were unsure whether import into the chloroplast had actually occurred or if there were other reasons for the specific labelling. Interestingly, no labelling could be detected in the expected location of the apoplastic space or in the golgi apparatus or secretory vesicles.

WO 94/4678 (Casterman et al) describes the isolation of immunoglobulins from Camelids. These immunoglobulins, hereinafter "heavy chain immunoglobulins" have a characteristic distinct structure in that they are naturally devoid of light chains, with the antigen-binding sites being contained entirely within the heavy chain variable domains. This in turn leads to a characteristic structure for the heavy chains of these immunoglobulins, because the variable domain has no interaction sites for a light chain and a complete antigen-binding site has to be formed with no contribution from the hypervariable loops of a light chain variable domain. The heavy chain variable fragments (HCV) of these immunoglobulins are often referred to as VHH fragments. Such immunoglobulins or fragments thereof, show the functional properties of conventional, four chain, immunoglobulins but by virtue of their simplified structure offer advantages in preparation and use.

There are no reports in the literature of correctly targeted expression in plants of heavy chain immunoglobulins or fragments thereof. A report has appeared in a PhD thesis (Vu, 1999, Vrije Universiteit, Brussels, Belgium) of expression of a heavy chain variable domain in fusion with a chloroplast targeting peptide in *Nicotiana tabacum.* However, targeting to the chloroplast appears to have failed and the subcelluar localisation of the antibody fragments (which remained as a fusion protein) was not established.

There remains a continuing need for the development of improved methods for the production of antibodies and fragments thereof in plants, with the aim of providing methods suitable for economical large scale production for protecting plants from pathogens and for modulating their metabolism.

### SUMMARY OF THE INVENTION

Accordingly, in a first aspect the invention provides a method for modifying a plant to produce an antibody or an active fragment or derivative thereof in a desired cellular compartment, comprising introducing into a plant a DNA sequence encoding a heavy chain immunoglobulin or an active fragment or derivative thereof, or a sequence encoding a protein functionally equivalent thereto, said DNA sequence being operably linked to one or more promoters and provided, as appropriate, with an additional sequence encoding a peptide sequence capable of targeting said antibody or fragment or derivative thereof to said desired cellular compartment.

Also provided is a method for preparing a heavy chain immunoglobulin or an active fragment or derivative thereof comprising extracting said immunoglobulin or fragment or derivative thereof from a plant modified according to the first aspect of the invention.

In an alternative aspect, the invention provides the use of a DNA sequence encoding a heavy chain immunoglobulin or an active fragment or derivative thereof, or a sequence encoding a protein functionally equivalent thereto, to cause a plant to produce said antibody or fragment or derivative thereof or protein functionally equivalent thereto in a desired cellular compartment.

Also provided is a modified plant having, in a desired cellular compartment, enhanced levels of heavy chain immunoglobulins or active fragments or derivatives thereof, or proteins functionally equivalent thereto, particularly compared to equivalent but unmodified plants.

Seeds, fruits and progeny of such plants and hybrids are included within the invention.

Food products such as sauces, dressings, tomato products such ketchups, meals, juices and soups, comprising a plant or part thereof according to the invention are also provided.

Also provided are skin and hair protective compositions and pharmaceutical compositions comprising a plant or part thereof according to the invention.

As used herein, "plant" means a whole plant or part thereof, or a plant cell or group of plant cells, or an extract thereof. The invention is particularly directed at transforming whole plants and the use of the whole plant or significant parts thereof.

The term "antibody" refers to an immunoglobulin which may be derived from natural sources or synthetically produced, in whole or in part. An "antibody fragment", alternatively an "active fragment", is a portion of a complete antibody or immunoglobulin which retains the ability to exhibit at least part, and preferably all, of the antigen binding activity.

A "heavy chain immunoglobulin" is an immunoglobulin naturally devoid of any variable light chain domains but which is capable of specifically combining with an antigen. The antigen-binding capacity and specificity is located exclusively in the immunoglobulin heavy chains, more specifically in the heavy chain variable domains.

The "sequence encoding the heavy chain immunoglobulin or an active fragment or derivative thereof" encompasses a genomic or cDNA clone or a sequence which in proper reading frame encodes an amino acid sequence which is functionally equivalent to the amino acid sequence of the protein encoded by the genomic or cDNA clone. By "functionally equivalent" is meant any protein or fragment or derivative thereof which has the same or similar antigen-binding properties, said antigen-binding capacity being located in a single binding domain. It should be understood, however, that isolated VH domains of conventional antibodies are not included within the scope of the invention. Similarity in functionality can be evaluated by routine screening assays, for example, by assaying the binding affinity of the immunoglobulins produced upon expression in plants.

A "functionally equivalent" derivative may be characterised by an insertion, deletion or substitution of one or more amino acid residues in the sequence of the heavy chain immunoglobulin or active fragment thereof and includes within its scope fusion molecules. Such derivatives may readily be made by using conventional techniques well known in the art such as peptide synthesis techniques or recombinant DNA manipulation of a gene encoding a heavy chain immunoglobulin by site directed mutagenesis.

It will be appreciated that immunoglobulins or fragments or derivatives thereof modified to enable them to function as binding domains in the same way as immunoglobulins naturally devoid of light chain domains ("heavy chain immunoglobulins") may also suitably be used according to the invention.

A "gene" is a DNA sequence encoding a protein, including modified or synthetic DNA sequences or naturally occurring sequences encoding a protein, and excluding the 5' sequence which drives the initiation of transcription.

"Operably linked to one or more promoters" means the gene, or DNA sequence, is positioned or connected to the promoter in such a way to ensure its functioning. The promoter is any sequence sufficient to allow the DNA to be transcribed. After the gene and promoter sequences are joined, upon activation of the promoter, the gene will be expressed.

An "equivalent, unmodified" plant is a plant which has a substantially identical genotype to a modified plant of the invention excepting the introduced sequences present in the plant of the invention.

A "construct" is a polynucleotide comprising nucleic acid sequences not normally associated in nature.

A "cellular compartment" is a volume or structure separated from the remainder of the cell by at least one membrane. For the purposes of the invention, this also includes the apoplastic space between cells. A "targeting peptide" or "transit peptide" is a polypeptide sequence which, when appended to the appropriate end of a protein sequence is capable of directing the import of said protein into a specific cellular compartment.

### BRIEF DESCRIPTION OF THE DRAWINGS

The present invention may be more fully understood by reference to the following description, when read together with the accompanying drawings in which:
- Figure 1: shows the nucleotide sequence (SEQ. ID. No. 1) and corresponding protein sequence (SEQ. ID. No. 2) of a fragment encoding the heavy chain variable domain of an anti-RR6 antibody (denoted HCV33) from a llama, with an attached peptide linker group (denoted myc) (RR6 is an azo-dye, available from ICI; myc is a peptide comprising the sequence EQKLISEEDLN, SEQ. ID. NO. 3).
- Figure 2: shows a specificity ELISA assay (A) and SDS-PAGE analysis (B) of whole cell extract of a tobacco plant transformed (Example 4) with the plant expression vector pPV.8 - HCV33-myc (of Example 1). The positive control was at a concentration of 250ng/ml. For the ELISA assay, 50µl of plant extract was used. Total protein concentration in the plant extract was 2mg/ml.
- Figure 3: shows a specificity ELISA assay (A) and SDS-PAGE analysis (B) of whole cell extracts of a tobacco plant transformed (Example 5) with the expression vector pPV.8-GBSS-HCV33-myc (of Example 1). The positive control was at a concentration of 20µg/ml. Total protein concentration in the plant extract was 1-2mg/ml.
- Figure 4: shows the results of immuno-histology studies, as reported in Example 5, on a tobacco plant transformed with the expression vector pPV.8-GBSS-HCV33-myc (of Example 1), confirming that HCV33 fragment is localised in the chloroplasts.
- Figure 5: shows the amount of active antibody present in fruit columella extracts of a tomato plant transformed (Example 5) with the plant expression vector pPV.8-GBSS-HCV33-myc (of Example 1), expressed as % of total soluble protein, as determined via direct binding ELISA.
- Figure 6: shows the nucleotide sequence (SEQ. ID. No. 4) and corresponding protein sequence (SEQ. ID. No. 5) of a fragment encoding the heavy chain variable domain of an anti-potato SBEII antibody (denoted Clonel) with attached peptide linker groups denoted His6 (His-His-His-His-His-His) and myc (as described in Figure 1). (Potato SBEII is a starch branching enzyme also denoted SBE A; Jobling et. *al.,* Plant Journal, 18, 163-171 1999).
- Figure 7: shows the amount of active antibody present in whole leaf extract of a tobacco plant transformed (Example 5) with the plant expression vector pSJ35-CERV-GBSS-Clonel-His6-myc (of Example 1), expressed as % of total soluble protein, as determined via direct binding ELISA.
- Figure 8: shows the results of immuno-histology studies as reported in Example 6 on a tobacco plant transformed with the expression vector pSJ.34-2x355-GBSS-scFv3299-hydrophilII (of Example 1). It is clear that plastid formation/function is disrupted.
- Figure 9: shows the amount of active antibody present in leaf extract of tobacco plants transformed (Example 7) with the plant expression vector pPV.8-PRla-HCV33-myc (of Example 1), expressed as % of total soluble protein, as determined via direct binding ELISA.
- Figure 10: shows a specificity ELISA assay (A) and SDS-PAGE/western blot analysis (B) of whole cell extracts of tobacco plants transformed (Example 8) with two plant expression vectors, namely pPV.8-PRIa-HCV33-myc-SEKDEL and pPV.8-GBSS-HCV33-myc-SEKDEL (of Example 1). Total protein concentration in the plant extract was 1-2mg/ml.
- Figure 11: shows the results of immuno-histology studies as reported in Example 8 on a tobacco plant transformed with the expression vector pPV.8-PRIa-HCV33-myc-SEKDEL (of Example 1) confirming that the HCV33 material, directed to the secretory pathway using the PRIa leader sequence, and retained in the endoplasmic reticulum by the C-terminal tag SEKDEL.
- Figure 12: shows the nucleotide sequence (SEQ. ID. No. 6) and corresponding protein sequence (SEQ. ID. No. 7) of a fragment encoding the heavy chain variable domain of an anti-potato SBEII antibody (denoted Clone46) with attached peptide linker groups denoted His6 and myc (as described in Figure 1).
- Figure 13: shows that the SBE (starch branching enzyme) activity of whole leaf extracts of 30 tobacco plants independently transformed (Example 9) with the plant expression vector pSJ.35-CERV-GBSS-Clone46-His6-myc (of Example 1) is significantly less than that of plants transformed with pSJ.35-CERV-GBSS-HCV33-myc (of Example 1).
- Figure 14: shows the nucleotide sequence (SEQ. ID. No. 8) and corresponding protein sequence (SEQ. ID. No. 9) of a fragment encoding the heavy chain variable domain of an anti-GUS antibody (denoted Clone18) with attached peptide linker groups denoted His6 and myc (as described in Figure 1). (GUS is β-glucuronidase, available from Sigma.)
- Figure 15: shows the nucleotide sequence (SEQ. ID. No. 10) and corresponding protein sequence (SEQ. ID. No. 11) of a fragment encoding the heavy chain variable domain of an anti-GUS antibody (denoted Clone18) with an endoplasmic retention signal (SEKDEL) attached peptide linker groups denoted His6 and myc (as described in Figure 1).
- Figure 16: shows that the GUS (β-glucuronidase) activity of whole leaf extracts of 30 pCJ.102 (of Example 1) transformed tobacco plants independently retransformed (Example 10) with the plant expression vector pSJ.35-CERV-Clone18-His6-myc-SEKDEL (of Example 1) is significantly less than that of control pCJ.102 plants.
- Figure 17: shows (A) X-glu staining of pollen from a tobacco plant transformed (Example 11) with pSJ.35-Lat52-HCV33-myc-SEKDEL (of Example 1), and (B) X-glu staining of pollen from a tobacco plant transformed (Example 11) with pSJ.35-Lat52-Clone18-His6-myc-SEKDEL (of Example 1).
- Figure 18: shows the nucleotide sequence (SEQ. ID. No. 54) and corresponding protein sequence (SEQ. ID. No. 55) of a fragment encoding the heavy chain variable domain of an anti-RR6 antibody (denoted HCV33) linked to the non-classical heavy chain constant regions (denoted hinge-CH2-CH3).
- Figure 19: shows the nucleotide sequence (SEQ. ID. No. 56) and corresponding protein sequence (SEQ. ID. No. 57) of a fragment encoding the heavy chain variable domain of an anti-RR6 antibody (denoted HCV33) linked to the non-classical heavy chain constant regions (denoted Hinge-CH2-CH3) with an endoplasmic retention signal (SEKDEL).
- Figure 20: shows the amount of active antibody present in whole leaf extract of a tobacco plant transformed (Example 12) with the plant expression vector pPV.8-HCV33-IgG2b (of Example 12), expressed as % of total soluble protein as determined via direct binding ELISA.
- Figure 21: shows the amount of active antibody present in whole leaf, stem and root extract of three tobacco plants (from Figure 20) analysed 6 weeks post germination, expressed as % of total soluble protein as determined via direct binding ELISA (of Example 12).
- Figure 22: shows the amount of active antibody present in whole leaf extract of a tobacco plant transformed (Example 12) with the plant expression vector pPV.8-GBSS-HCV33-IgG2b (of Example 12), expressed as % of total soluble protein as determined via direct binding ELISA.
- Figure 23: shows the amount of active antibody present in whole leaf, stem and root extract of three tobacco plants (from Figure 22) analysed 6 weeks post germination, expressed as % of total soluble protein as determined via direct binding ELISA (of Example 12).
- Figure 24: shows the amount of active antibody present in whole leaf extract of a tobacco plant transformed (Example 12) with the plant expression vector pPV.8-PR1a-HCV33-IgG2b (of Example 12), expressed as % of total soluble protein as determined via direct binding ELISA.
- Figure 25: shows the amount of active antibody present in whole leaf, stem and root extract of three tobacco plants (from Figure 24) analysed 6 weeks post germination, expressed as % of total soluble protein as determined via direct binding ELISA (of Example 12).
- Figure 26: shows the amount of active antibody present in whole leaf extract of a tobacco plant transformed (Example 12) with the plant expression vector pPV.8-PR1a-HCV33-IgG2b-SEKDEL (of Example 12), expressed as % of total soluble protein as determined via direct binding ELISA.
- Figure 27: shows the amount of active antibody present in whole leaf, stem and root extract of three tobacco plants (from Figure 26) analysed 6 weeks post germination, expressed as % of total soluble protein as determined via direct binding ELISA (of Example 12).
- Figure 28: shows the nucleotide sequence (SEQ. ID. No. 66) and corresponding protein sequence (SEQ. ID. No. 67) of a fragment encoding the heavy chain variable domain of an anti-potato SBEII antibody (denoted Clone68) with attached peptide linker groups denoted His6 and myc (as described in Figure 1).
- Figure 29: shows the amount of active antibody present in whole leaf extract of a tobacco plant transformed (Example 13) with the plant expression vector pSJ35-CERV-GBSS-Clone68-His6-myc (of Example 13) expressed as OD reading as compared to the purified and quantified *E. coli* clone68 standard.

### DETAILED DESCRIPTION OF THE INVENTION

The present invention is based on the finding that immunologically active immunoglobulins, or active fragments or derivatives thereof, which are devoid of light chain variable domains, can advantageously be expressed at high level and successfully targeted to specific cellular locations in plant cells, with retention of binding activity and no adverse effects on plant growth or morphology.

In particular, it has been found that the method of the invention can be used to produce significant amounts of whole heavy chain immunoglobulins, with retention of binding activity, in the major compartments of the plant cell, namely plastid, cytoplasm, apoplast and endoplasmic reticulum. Selfing of transformed, antibody-expressing plants produced a new generation of plants, many of which displayed an ability to accumulate higher levels of antibody than the parent plants. The amounts of antibody were found to be markedly greater than those typically achieved when conventional antibodies are expressed in plants. The accumulation of high levels of functional antibody in the cytoplasm was a particularly unexpected result, since conventional antibodies have generally been found to be produced only in very small amounts in this location, probably owing to the problem of forming the disulphide bonds needed for structural stability in a reducing environment.

The method of the invention was similarly found to be capable of producing VHH fragments in significant amounts in these different compartments. This was the case for several different VHH fragments, in contrast to the situation with fragments based on conventional antibodies, such as scFv, where the results have been found to be very unpredictable. Importantly, it was found that the accumulation of VHH fragments in any compartment of the cell resulted in no discernible deleterious effect on the plant; again this contrasts with results obtained with scFv fragments, where expression levels were found to be unstable and accumulation of the fragment in the plastids was found to affect adversely the structure and function of these organelles.

By means of the invention, antibodies and active fragments thereof, having specificity for a target antigen may conveniently be prepared by a method which is readily amenable to industrial-scale production. Further, the ability to target the antibodies to any chosen cell compartment makes it possible to use the method of the invention to modulate the activity of specific enzymes irrespective of where in the cell the enzyme is found.

In order to modify the function of an intracellular protein, the antibody or fragment or derivative thereof must be present and active in those compartments of the host plant cell where the target protein is active. In cases where this is the cytoplasm, the reducing environment inhibits the formation of disulphide links in the antibody and so generally antibodies would not be expected to be active in the cytoplasm. Expression of classical antibodies, or fragments thereof, in the cytoplasm does not generally give rise to functional actively, for example. Similarly, expression of scFv fragments in plastids generally leads to unstable deformed plastids. The targeted expression of active antibodies or fragments or derivatives thereof in compartments of the host plant cell, especially in the cytoplasm, therefore represents a particular advantage of the method of the invention.

The present inventors have found that even when the levels of expression of heavy chain immunoglobulin or fragment or derivative thereof in the plant cell are low or undetectable by conventional protein chemical methods such as in the case of expression in the cytoplasm, a measurable effect on enzyme activity may still be achieved. The ability to modulate plant enzyme activity without accumulating antibodies in large amounts may be beneficial in some circumstances, for example, in minimising potential deleterious effects on the growth and health of the plant.

The heavy chain immunoglobulins (or fragments or derivatives thereof) to be expressed in accordance to the invention are, or are derived from, immunoglobulins that are naturally devoid of light chains, their antigen binding sites being contained entirely within the heavy chain variable domains. Especially preferred are immunoglobulins or fragments or derivatives thereof which are obtainable from Camelids as described, for example, in WO 94/4678 above. Heavy chain variable domains of immunoglobulins naturally devoid of light chains are particularly preferred for use in the present invention.

Preferred immunoglobulins for use in the invention are obtainable from *Camelids* especially from *Lamas* (for example *Lama glama, Lama vicugna* or *Lama Paccos)* or from *Camelus* (for example *Camelus dromedarius* or *Camelus bactrianus).*

Conveniently, a functionally equivalent protein shows at least 50% similarity to the respective amino acid sequence, preferably at least 70% similarity, more preferably at least 80%, most preferably 90-100% similarity to the respective amino acid sequence as determined by techniques well known in the art.

The invention also provides for the expression of complex derivative molecules comprising an immunoglobulin or fragment thereof as described above, associated or otherwise connected to one or more other molecules, for example, an enzyme or a further heavy chain variable domain.

The invention provides specifically for the targeting of expressed antibodies, or fragment or derivatives thereof, to specific cellular compartments. Where the desired compartment is the cytoplasm, this is the default location when no specific targeting sequence is appended to the antibody. For targeting to other compartments, sequences encoding appropriate targeting signals are fused to the sequence encoding the antibody.

Where it is desired to target an antibody to the chloroplast, a sequence encoding an appropriate transit peptide is provided, such that said peptide will be fused at the N-terminus of the expressed antibody and subsequently removed upon passage into the organelle. Any chloroplast-specific transit peptide may be used together, optionally, with a few (1-3) residues from the N-terminus of the mature protein to which said peptide is fused in nature. A number of such sequences, proven to be capable of directing successful targeting of heterologous proteins, are well known to those skilled in the art. Particularly preferred is the GBSS transit peptide sequence from potato (van der Leij et al (1991) Mol. Gen. Genet., 228, 240-248).

Where it is desired to direct secretion of an antibody into the apoplastic space, a sequence encoding an appropriate signal peptide is provided, such that said peptide will be fused at the N-terminus of the expressed antibody and subsequently removed upon passage into the endoplasmic reticulum, en route to secretion. Any signal peptide may be used, including a signal peptide that would naturally direct secretion of an antibody, or other signal peptides derived from animal, plant or microbial secreted or ER-localised proteins. Many such sequences are well known to those skilled in the art. Particularly preferred is the PRIa leader sequence (Firek et al; Plant Mol. Biol, 23 861-870 (1993)).

Where it is desired to direct an antibody to the endoplasmic reticulum, and to retain it there, a signal sequence is provided, as above, and additionally an ER-retention signal is provided. Conveniently, this takes the form of a C-terminal polypeptide extension comprising the sequence Lys-Asp-Glu-Leu (KDEL). Alternatively, the KDEL sequence may be introduced by mutation of the residues at the C-terminal end of the antibody, or fragment of derivative thereof.

It will be appreciated that the invention extends to any plant which is amenable to transformation. Suitable plants include tobacco, peas, potatoes, spinach, tomato and tea.

The DNA sequences of interest will preferably be operably linked (that is, positioned to ensure the functioning of) to one or more suitable promoters which allow the DNA to be transcribed. Suitable promoters, which may be homologous or heterologous to the gene, useful for expression in plants are well known in art, as described, for example, in Weising et al, (1988), Ann. Rev. Genetics, 22, 421-477). Promoters for use according to the invention may be inducible, constitutive or tissue-specific or have various combinations of such characteristics. Useful promoters include, but are not limited to constitutive promoters such as carnation etched ring virus (CERV), cauliflower mosaic virus (CaMV) 35S promoter, or more particularly the double enhanced cauliflower mosaic virus promoter, comprising two CaMV 35S promoters in tandem (referred to as a "Double 35S" promoter).

It may be desirable to use a tissue-specific or developmentally regulated promoter instead of a constitutive promoter in certain circumstances. A tissue-specific promoter allows for expression of immunoglobulins in certain tissues. Suitable fruit-specific promoters include the tomato E8 promoter (Deikman et al, (1988), EMBO J, 7, 3315-3320), 2A11 (Van Haaren et al, Plant Mol Biol, 21, 625-640), E4 (Cordes et al, (1989), Plant Cell, 1, 1025-1034) and PG (Bird et al, (1988), Plant Mol. Biol., 11, 651-662,) Nicholass et al, (1995), Plant Molecular Biology, 28, 423-435.

The invention provides in a further aspect an expression cassette comprising as operably linked components in the 5'-3' direction of transcription, a promoter functional in a plant cell, one or more DNA sequences encoding a heavy chain immunoglobulin or an active fragment or derivative thereof or one or more sequences encoding a protein functionally equivalent thereto and a transcriptional and translational termination regulatory region functional in a plant cell.

The promoter and termination regulatory regions will be functional in the host plant cell and may be heterologous (that is, not naturally occurring in the host plant) or homologous (derived from the plant host species) to the plant cell and the DNA sequence. Suitable promoters which may be used are described above.

The termination regulatory region may be derived from the 3' region of the gene from which the promoter was obtained or from another gene. Suitable termination regions which may be used are well known in the art and include *Agrobacterium tumefaciens nopaline synthase terminator (Tnos), Agrobacterium tumefaciens mannopine synthase terminator (Tmas)* and the *CaMV 35S terminator (T35S).* Particularly preferred termination regions for use according to the invention include the tobacco ribulose bisphosphate carboxylase small subunit termination region (TrbcS) or the *Tnos* termination region.

Such DNA constructs may suitably be screened for activity by transformation into a host plant via *Agrobacterium* and screening for immunoglobulin levels.

In order to be able to select for plant cells that have integrated the construct, the expression construct may conveniently be joined to a marker which permits screening or selection, according to methods well known in the art.

Conveniently, the expression cassette according to the invention may be prepared by cloning the individual promoter/gene/ terminator units into a suitable cloning vector. Suitable cloning vectors are well known in the art, including such vectors as pUC (Norrander et al, (1983, Gene 26, 101-106), pEMBL (Dente et al (1983), Nucleic Acids Research, 11, 1645-1699), pBLUESCRIPT (available from Stratagene), pGEM (available from Promega) and pBR322 (Bolivar et al, (1977), Gene, 2, 95-113). Particularly useful cloning vectors are those based on the pUC series. The cloning vector allows the DNA to be amplified or manipulated, for example by joining sequences. The cloning sites are preferably in the form of a polylinker, that is a sequence containing multiple adjacent restriction sites, so as to allow flexibility in cloning.

Suitably, the nucleotide sequences for the genes may be extracted from the Genbank nucleotide database and searched for restriction enzymes that do not cut. These restriction sites may be added to the genes by conventional methods such as incorporating these sites in PCR primers or by sub-cloning.

Preferably the DNA construct according to the invention is comprised within a vector, most suitably an expression vector adapted for expression in an appropriate host (plant) cell. It will be appreciated that any vector which is capable of producing a recombinant plant comprising the introduced DNA sequence will be sufficient.

Suitable vectors are well known to those skilled in the art and are described in general technical references such as Pouwels et al, Cloning Vectors. A laboratory manual, Elsevier, Amsterdam (1986). Particularly suitable vectors include the Ti plasmid vectors.

Transformation techniques for introducing the DNA constructs according to the invention into host cells are well known in the art and include such methods as micro-injection, using polyethylene glycol, electroporation, or high velocity ballistic penetration. A preferred method for use according to the present invention relies on agrobacterium - mediated transformation.

After transformation of the plant cells or plant, those plant cells or plants into which the desired DNA has been incorporated may be selected by such methods as antibiotic resistance, herbicide resistance, tolerance to amino-acid analogues or using phenotypic markers.

Various assays, well known in the art, may be used to determine whether the plant cell shows the presence of the desired antibody, or fragment thereof. These include binding assay such as an ELISA or radio-immunoassay.

Plant cells transformed according to the invention may be grown in an appropriate culture or cultivated in soil or some other such suitable medium by methods well known in the art. It will be appreciated that the particular procedures adopted will vary depending on the plant species used, such variations being well within the knowledge of the average skilled practitioner.

These plant cells or plants may then conveniently be harvested and the desired antibody product processed using conventional extraction and purification techniques.

Seeds produced from modified plants containing the sequence coding for the desired antibody product can subsequently be grown to generate a progeny population of modified plants from which the desired product can be isolated.

Under some circumstances, it may be desirable to retain the antibody product with the plant rather than extracting and isolating the product. Where the modified plant or part thereof concerned is edible, for example, an antibody which has an effect on ingestion can conveniently be introduced in the form of a food product. In particular, edible plants or parts thereof, modified to incorporate an antibody or fragment or derivative thereof capable of binding a pre-selected antigen may conveniently be used in a method of passively immunising an animal, preferably a mammal and in particular a human, against said antigen. Particularly suitable antigens in this case will be pathogenic organisms or derivatives or parts thereof.

In addition to well-established application in the areas of diagnosis, therapy and purification, antibodies and fragments or derivatives thereof according to the present invention may suitably by used in methods to modify the properties of the plant in which they are produced. For example, the expression in a plant of functional anti-viral antibody can be useful in reducing pathogenicity and so provide a method of producing viral resistance. Alternatively, the introduction of antibodies into plants may be used to alter or interfere with plant metabolism by modulating the function of proteins present in the plant such as plant antigens and/or block phytohormones and metabolites.

The Invention will be further illustrated by means of the following examples which are provided by way of illustration only.

Techniques used for the modification and analysis of DNA materials were performed using standard procedures well known in the art, as described, for example, in Sambrook et al, "Molecular Cloning, A Laboratory Manual, Second Edition, Cold Spring Harbor, Laboratory Press 1989 (hereinafter Sambrook), unless otherwise indicated.

Restriction sites are underlined. HCV denotes heavy chain variable domain.

### EXAMPLES

### Example 1: Construction of HCV expression vectors

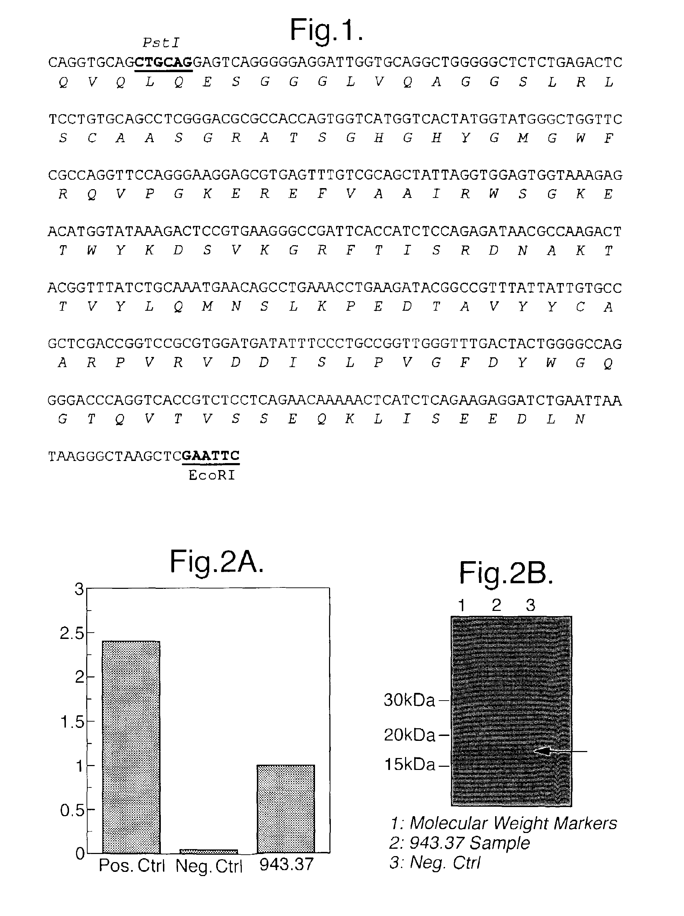

The construction of the HCV plant expression plasmids involved several cloning steps. The anti-RR6 HCV gene sequence was isolated from male llamas immunised with BSA-RR6 (as described in WO 99/23221). The sequence of the DNA fragment used in the following example (HCV33: SEQ. ID. No. 1) is given in Figure 1.

### pSJ.30

The plasmid pSJ.30 is a derivative of the binary vector pGPTV-KAN (Becker *et. al.,* Plant Mol. Biol. 20: 1195-1197, 1992) modified as follows: An approximately 750 bp (Sac I, T4 DNA polymerase blunted - Sal I) fragment of pJIT60 (Guerineau *et al., Plant Mol. Biol.* 18: 815-818, 1992) containing the duplicated cauliflower mosaic virus (CaMV) 35S promoter (denoted 2x35S; Cabb-JI strain, equivalent to nucleotides 7040 to 7376 duplicated upstream of 7040 to 7433, Frank *et. al., Cell* 21: 285-294, 1980) was cloned into the Hind III (klenow polymerase repaired) - Sal I sites of pGPTV-KAN to create pSJ.30.

### pPV.3

The shuttle vector pPV.3 was constructed by removing the HindIII/EcoRI multiple cloning site from pUC19 and replacing it by a synthetic DNA fragment, destroying the original EcoRI and HindIII sites and introducing a SgfI, HindIII, KpnI, EcoRI and XbaI restriction site. The new insert was constructed by annealing the synthetic oligonucleotides PCR.624 (SEQ. ID. No. 12) and PCR.625 (SEQ. ID. No. 13) (Table 1) yielding the insert sequence:

PCR.624

SgfI HindIII KpnI EcoRI XbaI
5′-AGCTGCGATCGCAAGCTTGGTACCGGGAATTCTCTAGA-3′
3′-CGCTAGCGTTCGAACCATGGCCCTTAAGAGATCTTTAA-5′

PCR.625

### pPV.5

The KpnI/EcoRI insert from pSJ.30 containing the 2x35S-promoter sequence upstream of the GUS gene, followed by the Nos terminator sequence was inserted into the KpnI/EcoRI opened pPV.3 vector yielding the intermediate vector pPV.5.

### pPV.5L

The GUS coding sequence was removed from the intermediate vector pPV.5 as a SalI/SacI fragment and replaced by a synthetic DNA fragment introducing a NcoI, NheI and MunI restriction site, while leaving the original SalI/SacI sites intact. The new insert was constructed by annealing the synthetic oligonucleotides PCR.626 (Seq. ID. No. 14) and PCR.627 (Seq. ID. No. 15) (Table 1) yielding the insert sequence:

PCR.626

SalI NcoI NheI MunI SacI
5′-TCGACCCATGGCCCGCTAGCCAATTGGAGCT-3′
3′-GGGTACCGGGCGATCGGTTAACC-5′

PCR.627

### pPV.5LN

The sequence present immediately 5' of the start codon ATG in this vector (CCACCATGG) was replaced by the plant Kozak sequence TAAACCATGG using PCR. Oligonucleotides PCR.640 (Seq. ID. No. 16) and PCR.641 (Seq. ID. No. 17) (Table 1) was used to amplify the 189 bp 3' fragment of the 2x355 promoter from vector pCP031 (Engelen et. al., Plant Mol. Biol., 26, 1701-1710, 1994), modifying the Kozak sequence via PCR.641. pCP031 and the amplified fragment (SEQ. ID. No. 18) were digested with HindIII/EcoRV and EcoRV/NcoI respectively and used to replace the promoter present in the intermediate vector pPV.5L. After verification of the sequence by sequencing with primer Seq. ID. No. 19 the vector pPV.SLN was used in subsequent cloning steps.

PCR.640/PCR.641 amplification product (SEQ. ID. No. 18)

PCR.640

CCACCCACGAGGGAACATCGTGGAAAAAGAAGACGTTCCAACCACGTCTTCAAAGCAAGT
GGTGGGTGCTCCCTTGTAGCACCTTTTTCTTCTGCAAGGTTGGTGCAGAAGTTTCGTTCA

EcoRV

GGATTGATGTGATATCTCCACTGACGTAAGGGATGACGCACAATCCCACTATCCTTCGCA
CCTAACTACACTATAGAGGTGACTGCATTCCCTACTGCTTGTTAGGGTGATAGGAAGCGT

NcoI

AGACCCTTCCTTTATATAAGGAAGTTCATTTCATTTGGAGAGGACCTCGAGTGTAAACCA

TCTGGGAAGGAAATATATTCCTTCAAGTAAAGTAAACCTCTCCTGGAGCTCACATTTGGT

PCR.641

TGGGAATTC

ACCCTTAAG

### pPV.5LN-PRIa-HCV33-myc

The PstI/EcoRI HCV33-myc fragment from pPIC.HCV33-myc (described in WO 99/23221 was inserted into the NcoI/MunI opened vector pPV.5LN together with a 105 bp NcoI/PstI fragment encoding the PRIa leader sequence allowing the in-frame fusion with the 5' framework-1 sequences of the HCV fragment.

The NcoI/PstI PRIa leader (Firek *et. al.*, Plant Mol. Biol., 23, 861-870, 1993) sequence (including the first 4 amino acids of the HCV fragment (QVQL)) was constructed by annealing the synthetic oligonucleotides PCR.293 (Seq. ID. No. 20), PCR.294 (Seq. ID. No. 21), PCR.295 (Seq. ID. No. 22), PCR.296 (Seq. ID. No. 23), and PCR.297 (Seq. ID. No. 24) (Table 1). The product of the annealing reaction was cloned as a HindIII/PstI fragment into pUC.19. After verification of the sequence of the synthetic gene fragment by sequencing with primers Seq. ID. No. 25, 26 the NcoI/PstI fragment was excised and used in subsequent cloning reactions.

### NcoI/PstI fragment encoding PRIA leader sequence (DNA Sequence Seq. ID. No. 27, Protein Sequence Seq. ID. No. 28)

NcoI
CCATGGGATTTGTTCTCTTTTCACAATTGCCTTCATTTCTTCTTGTCTCTACACTTCTCTT
GGTACCCTAAACAAGAGAAAAGTGTTAACGGAAGTAAAGAAGAACAGAGATGTGAAGAGAA
M G F V L F S Q L P S F L L V S T L L L

PstI
ATTCCTAGTAATATCCCACTCTTGCCGTGCCCAGGTGCAGCTGCAG
TAAGGATCATTATAGGGTGAGAACGGCACGGGTCCACGTCGACGTC
F L V I S H S C R A Q V Q L

### pPV.5LN-GBSS-HCV33-myc

The PstI/EcoRI HCV33-myc fragment from pPIC.HCV33-myc was inserted into the NcoI/MunI opened vector pPV.SLN together with a 252 bp NcoI/PstI fragment encoding the GBSS (including the first 4 amino acids of the HCV fragment (QVQL)) leader sequence allowing the in-frame fusion with the 5' framework-1 sequences of the HCV fragment.

The NcoI/PstI GBSS leader sequence was constructed by amplifying potato tuber cDNA (Sp. Desiree) with synthetic oligonucleotides 132 (Seq. ID. No. 29) and 133 (Seq. ID. No. 30), 134 (Seq. ID. No. 31) and 136 (Seq. ID. No. 32) (Table 1). The products of the amplification reaction were cloned as a NcoI/HpaI, HpaI/PstI fragment into pGEM5Zf(+) (Promega). After verification of the sequence by sequencing with primers Seq. ID. No. 26, 33 (Table 1) the NcoI/PstI was excised and used in subsequent cloning reactions.

### NcoI/PstI fragment encoding GBSS leader sequence (DNA Sequence Seq. ID. No. 34, Protein Sequence Seq. ID. No. 35)

NcoI
CCATGGCAAGCATCACAGCTTCACACCACTTTGTGTCAAGAAGCCAAACTTCACTAGACACCA
GGTACCGTTCGTAGTGTCGAAGTGTGGTGAAACACAGTTCTTCGGTTTGAAGTGATCTGTGGT
M A S I T A S H H F V S R S Q T S L D T

AATCAACCTTGTCACAGATAGGACTCAGGAACCATACTCTGACTCACAATGGTTTAAGGGCT
TTAGTTGGAACAGTGTCTATCCTGAGTCCTTGGTATGAGACTGAGTGTTACCAAATTCCCGA
K S T L S Q I G L R N H T L T H N G L R A

GTTAACAAACTTGATGGGCTCCAATCAAGAACTAATACTAAGGTAACACCCAAGATGGCATC
CAATTGTTTGAACTACCCGAGGTTAGTTCTTGATTATGATTCCATTGTGGGTTCTACCGTAG
V N K L D G L Q S R T N T K V T P K M A S

PstI
CAGAACTGAGACCAAGAGACCTGGATGCTCAGCTACCATTGTTTGTGGAAAACAGGTGCAGCTGCAG
GTCTTGACTCTGGTTCTCTGGACCTACGAGTCGATGGTAACAAACACCTTTTGTCCACGTCGACGTC
R T E T K R P G C S A T I V C G K Q V Q L Q

### pPV.5LN-HCV33-myc

The PstI/EcoRI HCV33-myc fragment from pPIC.HCV33-myc was inserted into the NcoI/MunI opened vector pPV.5LN together with a synthetic NcoI/PstI DNA fragment allowing the in-frame fusion with the ATG start codon. The synthetic linker was constructed by annealing the synthetic oligonucleotides PCR.652 (Seq. ID. No. 36) and PCR.653 (Seq. ID. No. 37) (Table 1) yielding the insert sequence:

PCR.652

NcoI PstI
5′-CATGCAGGTGCAGCTGCA-3′
3′-GTCCACGTCG-5′

PCR.653

### pPV.5LN-HCV33-myc-SEKDEL

The HindIII/BstEII Promoter-leader-HCV33 fragment inserts from pPVSLN-PRIa-HCV33-myc, pPVSLN-GBSS-HCV33-myc and pPV5LN-no leader-HCV33-myc was cloned into HindIII/MunI opened pPV5LN-PRIa-HCV33-myc vector together with a myc-SEKDEL encoding BstEII/EcoRI PCR fragment thus introducing the sequence SEKDEL at the C-terminal end of the myc-tag, thus yielding pPV.5LN-PRIa-HCV33-myc-SEKDEL, pPV.5LN-GBSS-HCV33-myc-SEKDEL and pPV.5LN-No leader-HCV33-myc-SEKDEL. The PCR fragment was generated using the synthetic oligonucleotides PCR.300 (Seq. ID. No. 38) and PCR.690 (Seq. ID. No. 39) (Table 1) using pPV.5LN-PRIa-HCV33-myc as a template.

### pSJ.34

The BamHI restriction site of the pGPTV-KAN expression vector containing the kanamycin resistance selectable marker gene (Becker *et. al.,* Plant Mol. Biol., 20, 1195-1197, 1992) was destroyed by restricting with BamHI followed by a Klenow reaction.

### pPV.8 plant transformation vectors

The HindIII/EcoRI inserts from vectors pPV.5LN-PRla-HCV33-myc, pPV.5LN-GBSS-HCV33-myc, pPV.5LN-No leader-HCV33-myc, pPV.5LN-PR1a-HCV33-myc-SEKDEL, pPV.5LN-GBSS-HCV33-myc-SEKDEL and pPV.5LN-No leader-HCV33-myc-SEKDEL were inserted into the HindIII/EcoRI vector fragment of pSJ.34, yielding pPV.8-GBSS-HCV33-myc, pPV.8-No leader-HCV33-myc, pPV.8-PR1a-HCV33-myc-SEKDEL, pPV.8-GBSS-HCV33-myc-SEKDEL and pPV.8-No leader-HCV33-myc-SEKDEL respectively.

### pPV.5LN-2x35S-GBSS-scFv3299-hydrophilII

The PstI/EcoRI (partial) fragment containing scFv3299-hydrophilII (also known as scFv.KC in patent filing WO 96/27612 and described in more detail in WO 99/27386)was inserted into the NcoI/MunI opened vector pPV.5LN together with a 252 bp NcoI/PstI fragment encoding the GBSS leader sequence (as described for pPV.5LN-GBSS-HCV33-myc) allowing the in-frame fusion with the 5' framework-1 sequences of the HCV fragment. By way of explanation, the sequence of scFv3299 is disclosed in W096/27612 (wherein it is described as FvKC-II), but with the BstEII-SacI linker fragment replaced with a BstEII-SacI fragment encoding a flexible linker yielding the sequence: TVTVSSGGGGSGGGGSGGGGSDIELT (Seq. ID. No. 40). The DNA sequence of the linker is described by Jackson et. *al.* (Selection of variants of antibodies and other protein molecules using phage display on the surface of bacteriophage fd. : in Protein engineering: A Practical Approach, eds. Rees, Sternberg and Wetzel, publ. IRL Press, Oxford, 1992)

### pSJ.34-2x35S-GBSS-scFv3299-hydrophilII

The HindIII/EcoRI 2x355 promoter-GBSS-scFV3299-hydrophilII fragment of pPV.5LN-2x35S-GBSS-scFv3299-hydrophilII was inserted into the HindIII/EcoRI vector fragment of pSJ.34.

### pSJ.89

A HindIII/SacI fragment containing (1x) 35S promoter-GUS.int (Vancanneyt *et. al.,* Mol. Gen. Genet, 220, 245-250, 1990) was inserted into the HindIII/SacI vector fragment of pSJ.34. This plant transformation vector with Kan resistance contains the 35S promoter driving the GUS.int gene.

### pSJ.103

The CERV promoter was isolated by PCR from infected *Dianthus barbatus* leaf material obtained from Dr Rene van der Lugt, Research Institute for Plant Protection (IPO-DLO), Binnehaven 5, P.O. Box 9060, NL-6700 GW, Wageningen, Netherlands, using the primers:

The approximately 380 bp fragment (corresponding to nucleotides 6737-7118 of Hull *et. al.,* EMBO Journal, 5, 3083-3090, 1986) was amplified with oligonucleotides CERV1 (Seq. ID. No. 41) and CERV2 (14 nucleotides downstream of the transcription start site) (Seq. ID. No. 42) (Table 1) and was cloned into the TA cloning vector pT7Blue (Invitrogen) with the promoter in the same orientation as the T7 promoter. Sequence analysis showed that the isolated CERV promoter differed from the published sequence at several positions (T-C at 6790, C-T at 6826, A-G at 6872 and T-A at 6729) the most significant of which was a small deletion in a polyA tract in the 5' untranslated leader (4A's vs. 9A's in the published sequence). These changes probably represent differences in virus isolates rather than PCR errors but these cannot be ruled out.

A HindIII - BamHI fragment from pSJ.103 was used as the source of the promoter in plant transformation vectors. Since the HindIII site was 13 nucleotides downstream of the CERV1 oligonucleotide, this sequence was not present in the promoter.

### pSJ.108

The HindIII/BamHI CERV promoter fragment of pSJ.103 was inserted into the HindIII/BamHI vector fragment of pSJ.89. This plant transformation vector with Kan resistance contains the CERV promoter driving the GUS.int gene.

### pPV.7

The HindIII/EcoRI insert from pSJ.108 containing the CERV promoter sequence upstream of the GUS gene, followed by the Nos terminator sequence was inserted into the HindIII/EcoRI opened pPV.3 vector yielding the intermediate vector pPV.7.

### pPV.7L

The GUS coding sequence was removed from the intermediate vector pPV.7 as a BamHI/SacI fragment and replaced by a synthetic DNA fragment introducing a NcoI, NheI and MunI restriction site, while leaving the original BamHI/SacI sites intact. The new insert was constructed by annealing the synthetic oligonucleotides PCR.628 (Seq. ID. No. 43) and PCR.629 (Seq. ID. No. 44) (Table 1) yielding the insert sequence:

PCR.628

BamHI NcoI NheI MunI SacI
5'-GATCCCATGGCCCGCTAGCCAATTGGAGCT-3'
3'-GGTACCGGGCGATCGGTTAACC-5'

PCR.629

### pPV.7LN

The sequence present immediately 5' of the start codon ATG in this vector (GATCCCATGG) was replaced by the plant Kozak sequence TAAACCATGG by replacing the BamHI/NheI fragment present in pPV.7L with a synthetic fragment constructed by annealing the synthetic oligonucleotides PCR.645 (Seq. ID. No. 45) and PCR.646 (Seq. ID. No. 46) (Table 1) yielding the vector pPV.7LN.

PCR.645
BamHI NcoI NheI
5′-GATCCACCTCGAGTGTAAACCATGGCCCG-3′
3′-GTGGAGCTCACATTTGGTACCGGGCGATC-5′
PCR.646

### pPV.7LN-GBSS-HCV33-myc

The PstI/MunI HCV33-myc fragment of pPIC.HCV33-myc was inserted into the NcoI/MunI opened vector pPV.7LN together with a 252 bp NcoI/PstI fragment encoding the GBSS leader sequence (as described for pPV.5LN-GBSS-HCV33-myc) allowing the in-frame fusion with the 5' framework-1 sequences of the HCV fragment.

### pPV.7LN-GBSS-Clonel-His6-myc

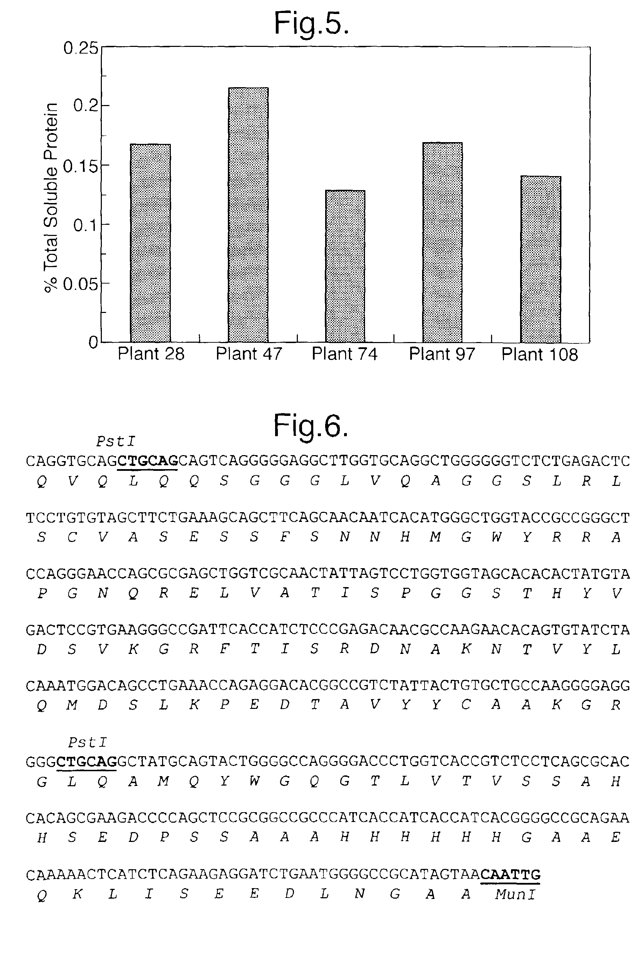

The Clone1-His6-myc sequence was amplified by PCR using oligonucleotides PCR.288 (Seq. ID. No. 47) and JWHCV2 (Seq. ID. No. 48) (Table 1) from pHEN.Clone1-His6-myc. The PstI (partial)/MunI fragment was inserted into the NcoI/MunI opened vector pPV.7LN together with a 252 bp NcoI/PstI fragment encoding the GBSS leader sequence (as described for pPV.SLN-GBSS-HCV33-myc) allowing the in-frame fusion with the 5' framework-1 sequences of the HCV fragment. The DNA sequence of clone 1 (Seq. ID. No 5, Figure 6) was then verified with sequencing primers, Seq. ID. No. 19,49 (Table 1.

### pPV.7LN-GBSS-Clone46-His6-myc

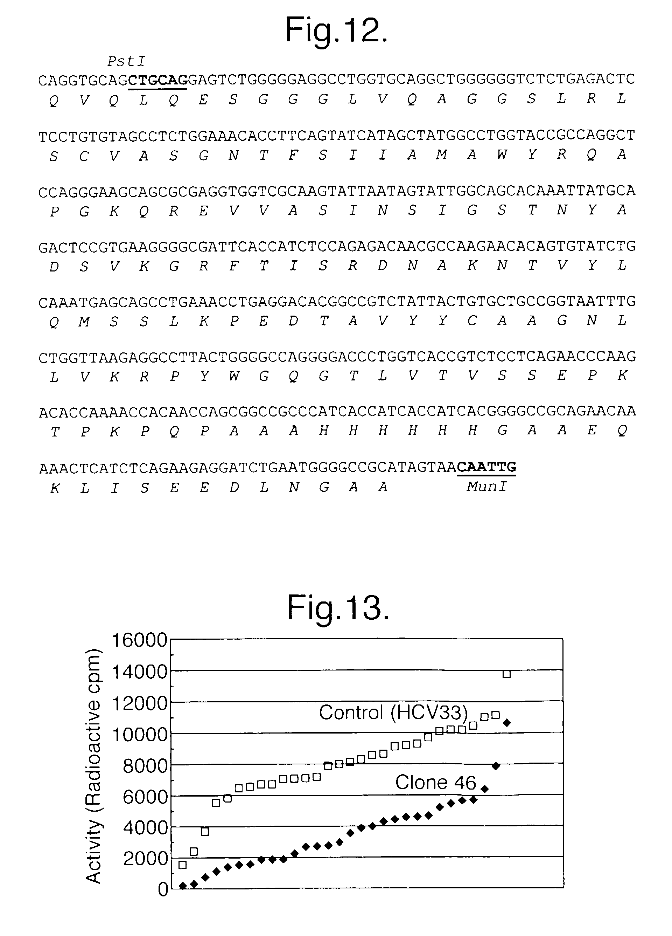

The Clone46-His6-myc sequence was amplified by PCR using oligonucleotides PCR.288 and JWHCV2 from pHEN.Clone46-His6-myc. The PstI/MunI fragment was inserted into the NcoI/MunI opened vector pPV.7LN together with a 252 bp NcoI/PstI fragment encoding the GBSS leader sequence (as described for pPV.5LN-GBSS-HCV33-myc) allowing the in-frame fusion with the 5' framework-1 sequences of the HCV fragment. The DNA sequence of clone 46 (Seq. ID. No. 6, Figure 12) was then verified by sequencing with primers, Seq. ID. No. 19,49 (Table 1).

### pPV.7LN-Clone18-His6-myc

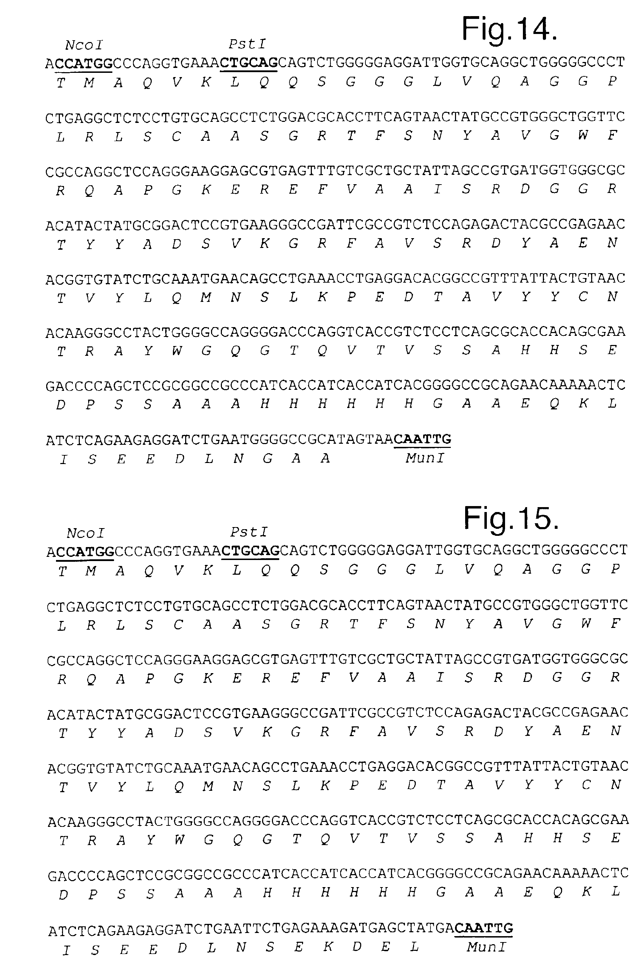

The Clone18-His6-myc sequence was amplified by PCR using oligonucleotides PCR.288 and JWHCV2 from pHEN.Clone18-His6-myc. The NcoI/MunI fragment was inserted into the NcoI/MunI opened vector pPV.7LN allowing the in-frame fusion with the 5' framework-1 sequences of the HCV fragment. The DNA sequence of clone 18 (Seq. ID. No. 8, Figure 14) was then verified by sequencing with primers, Seq. ID. No. 19,49 (Table 1).

### pPV.7LN-Clone18-His6-myc-SEKDEL

The Clone18-His6-myc sequence was amplified by PCR using oligonucleotides PCR.288 and JWHCV1 (Seq. ID. No. 50) (Table 1; to generate 3' SEKDEL endoplasmic retention signal) from pHEN.Clone18-His6-myc. The NcoI/MunI fragment was inserted into the NcoI/MunI opened vector pPV.7LN allowing the in-frame fusion with the 5' framework-1 sequences of the HCV fragment. The DNA sequence of Clone18-His6-myc-SEKDEL (Seq. ID. No. 10, Figure 15) was then verified by sequencing with primers, Seq. ID. No. 19,49 (Table 1).

### pSJ.35

The BamHI restriction site of the pGPTV-HYG expression vector containing the hygromycin resistance selectable marker gene (Becker *et. al.*, Plant Mol. Biol., 20, 1195-1197, 1992) was destroyed by restricting with BamHI followed by a Klenow reaction.

### pSJ.35-CERV plant transformation vectors

The HindIII/EcoRI inserts from vectors pPV.7LN-GBSS-HCV33-myc, pPV.7LN-GBSS-Clone1-His6-myc, pPV.7LN-GBSS-Clone46-His6-myc, pPV.7LN-Clone18-His6-myc and pPV.7LN-Clone18-His6-myc-SEKDEL were inserted into HindIII/EcoRI vector fragment of pSJ.35, yielding pSJ.35-CERV-GBSS-HCV33-myc, pSJ.35-CERV-GBSS-Clone1-His6-myc, pSJ.35-CERV-GBSS-Clone46-His6-myc, pSJ.35-CERV-Clone18-His6-myc and pSJ.35-CERV-Clone18-His6-myc-SEKDEL respectively.

### pCJ.102

Oligonucleotides 167 (Seq. ID. No. 51) and 168 (Seq. ID. No. 52) (Table 1) were used to amplify the 460bp 5' fragment of the GUS gene (from vector pSJ.34) and modifying the 5' end to create a NcoI site and thus modifying the second amino acid.
The PCR amplified fragment was digested with NcoI and EcoRV and inserted into HindIII/EcoRI vector fragment of pSJ.34 together with the HindIII/NcoI 2x35S promoter fragment of pPV.5LN and the EcoRV/EcoRI 3'GUS-Nos terminator fragment of pSJ.34, yielding the vector pCJ.102. The DNA sequence of the amplified fragment was verified by sequencing with primer Seq. ID. No. 53 (Table 1).

### pSJ.35-Lat52-Clone18-His6-myc

The NcoI/EcoRI Clone18-His6-myc-Nos fragment of pPV.7LN-Clone18-His6-myc was inserted into the SalI/EcoRI vector fragment of pSJ.35 together with the SalI/NcoI Lat52 promoter fragment of pLAT52-7 (Twell *et. al.,* Development, 109, 705-13, 1990).

### pSJ.35-Lat52-Clone18-His6-myc-SEKDEL

The NcoI/EcoRI Clone18-His6-myc-SEKDEL-Nos fragment of pPV.7LN-Clone18-His6-myc-SEKDEL was inserted into the SalI/EcoRI vector fragment of pSJ.35 together with the SalI/NcoI Lat52 promoter fragment of pLAT52-7 (Twell *et. al.,* Development, 109, 705-13, 1990).

### pSJ.35-Lat52-HCV33-myc-SEKDEL

The PstI/SacI HCV33-myc-SEKDEL fragment of pPV.8-GBSS-HCV33-myc-SEKDEL was inserted into the SalI/SacI vector fragment of pSJ.35 with the SalI/PstI Lat52 promoter-5' antibody gene fragment of pSJ.35-Lat52-Clone18-His6-myc.

### Example 2: Transformation and cultivation of Nicotiana Tabacum

Cells of the agrobacterium strain LBA4404 were transformed with the described plasmids using the freeze thaw method (Plant Molecular Biology Manual PMAN A3/7).

Seeds of Nicotiana Tabacum (var. Petit Havana SR1) were surface sterilised for 10 minutes in a solution of 10% sodium hypochlorate, rinsed 3 times in sterile distilled water and planted in Murashige & Skoog (MS) basal medium + 3% sucrose + 0.9% agar.

After 2 weeks growth seedlings were thinned to 2 per vessel; sterile plantlets were maintained by monthly shoot cuttings onto MS basal media +3% sucrose + 0.9% agar.

Discs were punched from leaves of these plants using a sterile cork borer, then incubated for 10 minutes in a culture of the agrobacterium strain LBA4404 containing the described plasmids which had been grown overnight in Lennox Broth (5g/l NaCl, Yeast Extract 10 g/l, 10 g/l Bacto Tryptone, 15g/l agar). The overnight culture was spun down for 10 minutes at 3000G and the growth media replaced by re-suspending the cells in MS Basal medium +3% sucrose.

Discs were removed from the culture and blotted dry on sterile filter paper before plating face down on a nurse culture plate of tobacco cells. This plate was prepared by adding 2ml of a cell suspension culture of Nicotiana Bethiama onto a petri dish containing 25ml of MS Salts, B5 vitamins (1mg/l Nicotinic acid, 1mg/lPyridoxine, 10mg/lThiamine, 100mg/l Inositol) +1mg/l 2-4 D, 0.2 mg/lBAP & 0.8%agar. The cells were swirled to cover the agar then covered with a sterile filter paper disc.

Infected leaf discs were incubated at 26°C in a light intensity of 2000 lux for 2 days, then removed from the nurse culture plates to selection media (MS Basal media +3% sucrose, 0.2mg/l IAA, 1mg/lBAP, 0.9%agar + cefotaxime 500mg/l and kanamycin 100 mg/l or hygromycin B 20mg/l). Plates were then incubated at 3000 lux, 26 C, 16 hours day/8 hour night and transferred every 2 weeks to fresh media (as previously). Shoots that appeared from the cut edges of the discs were removed to MS Basal media + 3% sucrose, cefotaxime 500mg/l and kanamycin 100mg/l or hygromycin B 20mg/l to root. Leaf samples were taken from rooted shoots for testing as described in Example 3, 4, 5,7,8 & 9.

### Example 3: Extraction and detection of HCV material

### Extraction of leaf tissue for analysis of anti-RR6 antibody (denoted HCV 33)

Leaf tissue was homogenised with Polytron probe in 2ml microcentrifuge tubes in the presence of 4ml extraction buffer (200mM Tris-HCl pH7.5, 5mM EDTA, 0.1% Tween 80, 1 mM PMSF {PhenylMethylSulfonylFluoride}) per 1gr leaf material. Solid material was removed from the homogenate by centrifugation (10 min 13,000g). The cleared supernatant was stored at -20°C. Total protein concentration was determined using the PIERCE BCA Protein Assay Reagent detection system.

### Extraction of tomato fruit tissue for analysis of anti-RR6 antibody (denoted HCV33)

Fruit were dissected into peel, flesh and columella (centre of fruit), frozen and ground with a mortar and pestle in liquid nitrogen. Extracts were prepared as for leaf tissue above.

### Extraction of leaf tissue for analysis of anti-potato

### SBEII antibodies (denoted Clone1 and Clone46)

Leaf tissue was homogenised with Polytron probe in 2ml microcentrifuge tubes in the presence of 4 ml SBE extraction buffer (100mM Tris-HCL pH7.5, 5mM EDTA, 2.5mM 1,4-dithiothreitol, 0.1% (w/v) sodium metabisulphite, 5% (v/v) glycerol, 2.5% (w/v) polyvinylpolypyrollidone, 1mM PMSF, 1 tablet Complete (Roche) per 50ml) per lgr fresh weight. Solid material was removed from the homogenate by centrifugation (10 min 13,000g). The cleared supernatant was stored at -20°C. Total protein concentration was determined using the BioRad Protein Assay Reagent detection system.

### Extraction of leaf tissue for analysis of anti-GUS antibody (denoted Clone18)

As described for analysis of anti-RR6 antibody, except GUS extraction buffer (50mM phosphate pH 7.0, 10mM EDTA, 0.1% Triton X-100, 1 mM DTT).

### Detection of RR6 binding activity

96 well ELISA plates (Greiner) were coated overnight at 4°C with 100µl per well of BSA-RR6 conjugate (10µg/ml) in 0.05 M NaCO₃ buffer pH9.5. Following three washes with tap water, the plates were incubated for 1 hour at 37°C with 250µl per well of a blocking solution of 3% Marvel in TBST (20mM Tris-HCL pH7.5, 140mM Nacl, 0.1% Tween 20). Test plant extracts and appropriate standards (purified, *P.pastoris* produced HCV33-myc standard) in duplicate were added to wells in a total volume of 100µl TBST, and the plates incubated at 4°C overnight. Unbound antibody fragment was removed by 6 washes with tap water. 100µl of the primary monoclonal anti-myc antibody No. 4111 (1µg/mL) in 1% Marvel/TBST were added to each well. Following incubation at 37°C for 1 hour, unbound antibody was removed by 6 washes with tap water and 100 µL of the secondary anti-mouse antibody conjugated alkaline phosphatase (1ug/mL) was added to each well. Following incubation at 37°C for 1 hour, unbound antibody was removed by 6 washes with tap water, and alkaline phosphatase activity was detected by adding 100µl of 1mg/ml pNPP in 1M diethanolamine/1mM MgCl₂. Plates were incubated at room temperature for up to 5 hours before reading at 410nM on a Dynex, MCX plate reader.

### Detection of SBEII binding activity

As for RR6 binding activity, except:

100µl/well of 5µg/ml recombinant potato SBEII was bound to the plate (Jobling *et al.,* Plant Journal, 18, 163-171, 1999) in PBS overnight at 4°C

Purified and quantified clone1-myc from E.coli was used as the standard.

Blocking buffer: 1% BSA in TBST, all incubations at room temperature.

100µl/well of 1:2,000 dilution of rabbit anti-llama IgG, as the primary detection antibody.

100µl/well of 1ug/mL goat anti-rabbit antibody conjugated alkaline phosphatase, as the secondary detection antibody.

### SDS-PAGE and Western blotting

Plant extracts were analysed on 10% bis-acrylamide gel using the Bio-Rad mini-Protean II system according to manufacturers instructions. 40µg samples and appropriate standards (purified *P.pastoris* produced HCV33-myc) were diluted in 50µl 2xSDS-PAGE loading buffer (20% Glycerol, 20mM Tris-HCL pH 8.0, 0.5% SDS, 0.005% bromophenol blue). The samples were mixed, boiled (5 minutes) and centrifuged briefly before loading onto the gel. The gels were run at 100V for 1 hour in 1x running buffer (25mM Tris, 200mM glycine, 1% SDS).

Proteins were transferred onto nylon membranes (Millipore) by semi dry blotting at 25V, 80mA for 45 minutes in semi-dry blotting buffer (50mM Tris, 40mM glycine, 0.04% SDS, 20% methanol). The membranes were then dried overnight at room temperature. The membranes were incubated in 3% Marvel, TBST for 10 minutes then in 1ug/mL mouse anti-myc antibody in 1% Marvel, TBST for 60 minutes at room temperature with constant agitation. Unbound antibody was removed by washing 6 times in TBST at room temperature with constant agitation. The blot was then incubated with the secondary antibody (rabbit anti-mouse IgG conjugated to alkaline phophatase) in 1% Marvel, TBST for 60 minutes at room temperature with constant agitation. Unbound antibody was removed by washing 6 times in TBST at room temperature with constant agitation. Bound antibody was detected by incubating the filters in 10mL of 30mg/mL NBT and 15mg/ml BCIP. Washing the filters with water stopped the colour reaction.

### EM studies

### Step 1: Sample preparation:

Leaf samples were fixed using 1% Paraformaldehyde+ 0.05% glutaraldehyde in 0.05M sodium phosphate buffer pH6.8, for 4hrs at 4°C, and then washed in phosphate buffer overnight at 4°C. Samples were embedded by first dehydrating the samples through alcohol, 50, 70, 90% (15mins) and finally absolute alcohol (2x30mins). They were then placed in hydrophilic resin [6 parts LRGold resin +_4 parts GMA (low acid) +0.1% benzoin ethyl ether (UV activator). Resin was changed several times over period of three days. Finally, samples were embedded, using freshly prepared resin, in flat bottomed plastic moulds, allowing the resin to polymerise for 24hrs at room temperature, under nitrogen gas, using UV light (360nm).

### Step 2: Staining:

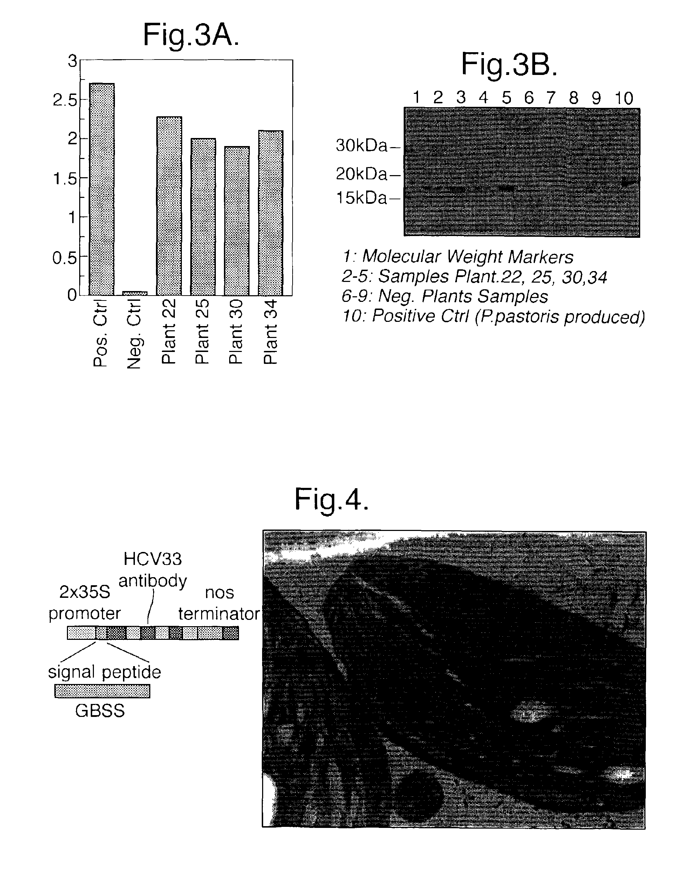

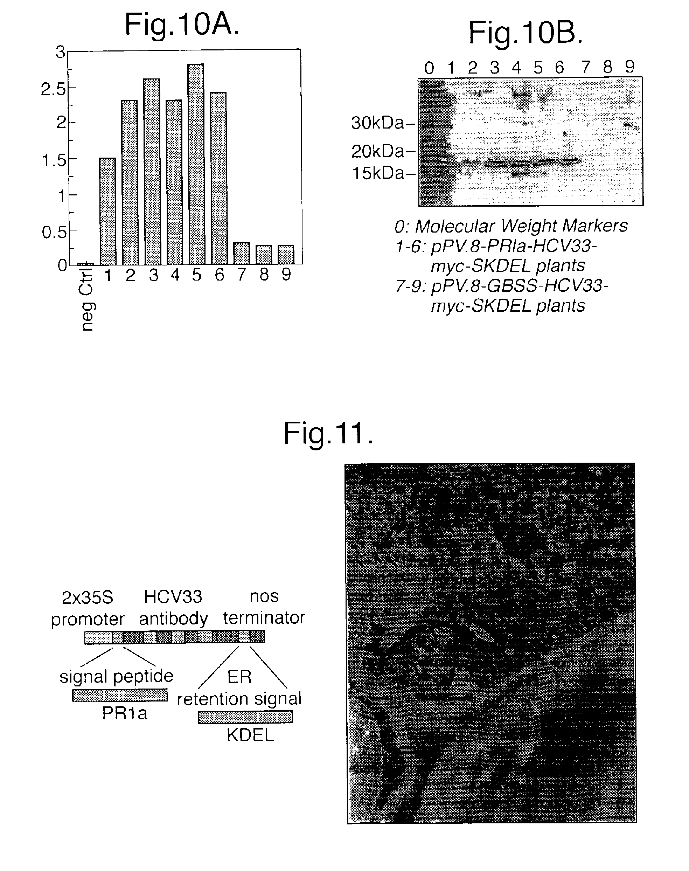

Ultrathin leaf tissue sections were collected on collodion (2% in amyl acetate) coated nickel e.m. grids. Grids were floated on 20µl aliquots of TBS/BSA [20mM Tris/HCL buffer pH7.6 + 0.23M NaCl +1 bovine serum albumin] for 20mins at room temperature, following by incubation with 20µl aliquots of primary antibody (anti-myc or anti-hydrophil II, 1µg/ml) in TBS/BSA +0.05% Tween 20 for 18hrs at room temperature. Grids were washed thoroughly using TBS. The primary antibody was detected by incubating the grids with 20µl aliquots of secondary antibody/gold conjugate diluted in TBS/BSA (Goat a Mouse IgG; 5nm diluted 1:200, 10nm diluted 1:100 20nm diluted 1:50) for 60mins at room temperature. The grids were washed thoroughly, using TBS followed by washes with deionised water and dried. Some grids were silver enhanced, prior to poststaining with 2% aqueous Uranyl Acetate, followed by Lead Citrate. Photographs were taken using a either a Jeol 100CX Mk2 transmission electron microscope at 60,000 magnification (Figure 4) or a Jeol 1220 transmission electron microscope at 40,000 magnification (Figure 11).

### Assay of SBE activity

All protein samples were diluted to a concentration of 1mg/mL using SBE extraction buffer. SBE was assayed using the phosphorylase a stimulation assay (Hawker *et al.,* Arch. Biochem. Biophys., 160, 530-551, 1974). The reaction was carried out at 30°C for 1h on 50µg protein in a final volume of 200µl and contained 100mM 2-(N-morpholino)ethanesulphonic acid (MES), 50mM [U-14C]glucose-1-phosphate (370MBq mole-1, ICN), 1.3U rabbit muscle phophorylase a (Sigma), 50µl plant extract at 1mg/ml protein. Glucose polymers were precipitated and washed as described by Hawker *et al.,* Arch. Biochem. Biophys., 160, 530-551 (1974). The final pellet was dissolved in DMSO and counted with ReadySafe-scintillation cocktail (Beckmann).

### Assay of GUS activity

All protein samples were diluted to a concentration of 1 mg/ml using GUS extraction buffer. 10µl samples were then added to 190µl of 1 x reaction buffer (50 mM phosphate pH 7.0, 0.1% Triton X-100, 1 mM DTT, 1 mM p-nitrophenyl β-D-glucuronide) contained in the well of a flat-bottomed microtitre plate. The reaction was left to proceed at 37°C until the wells turned visibly yellow. At this point, 80µl of stop solution (2.5M 2-amino-2-methyl propanediol) was added and the plate read at 410nm using a Dynex plate reader. GUS activity was calculated based on positive control wells to which known quantities of GUS were added.

### Histochemical staining for GUS activity

Tobacco pollen was stained for GUS activity in microtitre wells for up to 1 hour in 1 mM 5-bromo-4-chloro-3-indolyl β-D-glucuronic acid according to the method of Jefferson *et al.,* EMBO Journal 6: 3901-7, (1987). The buffer was modified by the use of histochemical staining buffer comprising 100 mM sodium phosphate pH 7.0, 10 mM EDTA, and 0.1% (v/v) Triton X-100 (Stomp, In: *Editorial comments,* Vol. 16, No.5. Cleveland: united State Biochemical, 1990). To minimalise spread of the stain to surrounding pollen grains, 5mM potassium ferricyanide was added to localise staining (Lojda, Histochemie 22: 347-61, 1970; Mascarenhas and Hamilton, Plant Journal 2: 405-8, 1992).

### Example 4: Expression of HCV antigen binding activity in the cytoplasm

Small samples of the leaves of 10 individual Tobacco plants transformed with the expression vector pPV.8-HCV33-myc were tested for the presence of anti-RR6 binding activity using ELISA. One of the tested samples showed low levels of RR6 binding activity. Detailed analysis of the anti-RR6 binding activity present in this clone (No. 943.37) using SDS-PAGE/Western blot analysis, ELISA and total protein concentration measurement showed that HCV33-myc was expressed as 0.001-0.002% of total protein present (Figure 2 and Table 2).

### Example 5: Expression of HCV antigen binding activity in plastids

Small samples of the leaves of 15 individual Tobacco plants transformed with the expression vector pPV.8-GBSS-HCV33-myc were tested for the presence of anti-RR6 binding activity using ELISA. Seven of the tested samples showed high levels of RR6 binding activity. Detailed analysis of the anti-RR6 binding activity present in these clones using SDS-PAGE/Western blot analysis, ELISA and total protein concentration measurement showed that HCV33-myc was expressed as 0.5-1.0% of total soluble protein present (Figure 3 and Table 2). From the Western blot it can be concluded that the construct is processed correctly and that the GBSS leader is removed from the HCV fragment. Localisation of the HCV33 material in the chloroplasts was confirmed via immuno-histology studies (Figure 4).

The fruit of 10 individual tomato plants transformed with the expression vector pPV.8-GBSS-HCV33-myc were picked 15 days post-breaker (red stage), dissected and tested for the presence of anti-RR6 binding activity using ELISA. 5 of tested fruit expressed levels of RR6-binding activity which, by comparison to a standard curve of purified HCV33 binding to RR6, were over 0.1% (one was over 0.2%) of total soluble protein (Figure 5 and Table 2).

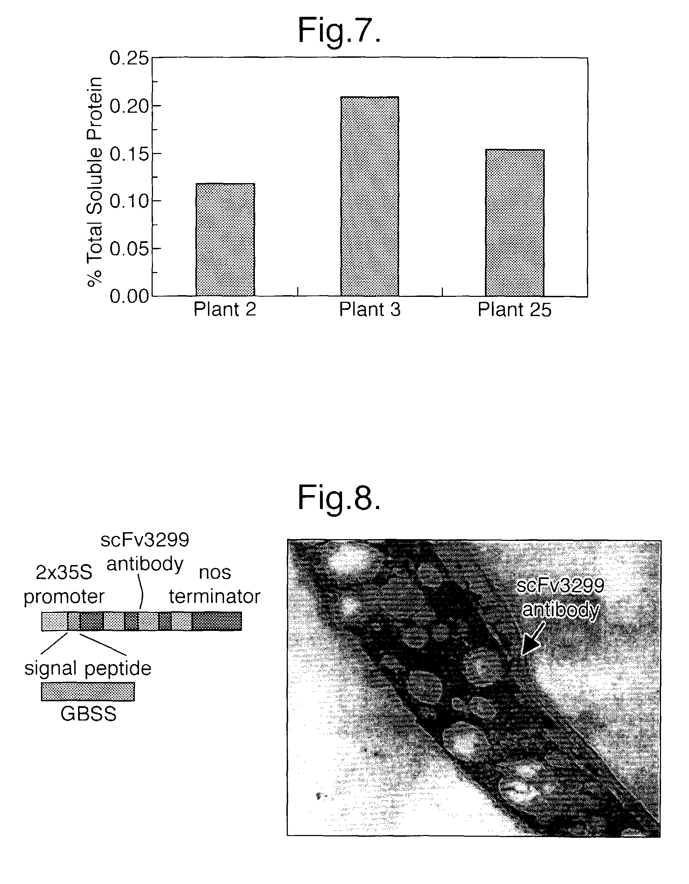

Similar high levels of expression were obtained with an HCV antibody of different specificity. Small samples of the leaves of 30 individual tobacco plants transformed with the expression vector pSJ.35-CERV-GBSS-Clonel-His6-myc were tested for the presence of anti-SBEII binding activity using ELISA. 3 of the tested plants expressed levels of SBEII-binding activity over 0.1% total soluble protein (Figure 7 and Table 2).

### Example 6: Disruption of plastid by scFv expression

Tobacco plants were transformed with expression vector pSJ34-2x35S-GBSS-scFv3299-hydrophilII. After initial detection of high expression of hCG binding activity, these levels were lost - expression was unstable (not shown). Furthermore, immuno-histology studies (Figure 8) suggested that scFv expression had an adverse affect on plastid structure and function, perhaps due to the aggregation of multiple scFv molecules at the plastid membrane.

### Example 7: Expression of HCV antigen binding activity in the apoplastic space

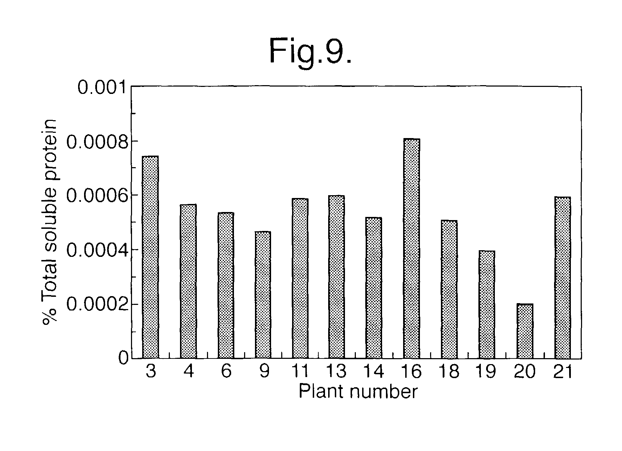

Small samples of the leaves of 21 individual Tobacco plants transformed with the expression vector pPV.8-PRIa-HCV33-myc were tested for the presence of anti-RR6 binding activity using ELISA. 12 of the tested samples showed low levels of RR6 binding activity. Detailed analysis of the anti-RR6 binding activity present using showed that HCV33-myc was expressed as 0.0002-0.00074% of total protein present (Figure 9 and Table 2).

### Example 8: Expression of HCV33 containing the ER retention signal SEKDEL

Small samples of the leaves of 22, 23 and 12 individual Tobacco plants transformed with the expression vectors pPV.8-no leader-HCV33-myc-SEKDEL, pPV.8-PRIa-HCV33-myc-SEKDEL, pPV.8-GBSS-HCV33-myc-SEKDEL respectively were tested for the presence of anti-RR6 binding activity using ELISA. None, 16 and 3 of the tested samples respectively, showed medium to high levels of RR6 binding activity. Detailed analysis of the anti-RR6 binding activity present in these clones using SDS-PAGE/Western blot analysis, ELISA and total protein concentration measurement showed that HCV33-myc was expressed as 0, 0.4 and 0.05% of total protein present respectively (Figure 10 and Table 2). Localisation of the HCV33 material, which was directed to the secretory pathway by using the PRIa leader sequence, in the ER was confirmed using immuno-histology studies (Figure 11).

### Example 9: Effect of neutralising HCV expression on SBE activity in tobacco plants

Samples of the leaves of 30 individual tobacco plants transformed with expression vector pSJ.35-CERV-GBSS-Clone46-His6-myc were assayed for SBE activity. Compared to the effect of the HCV33 anti-RR6 antibody in 30 control plants transformed with expression vector pSJ.35-CERV-GBSS-HCV33-myc, the specific anti-potato SBEII antibody Clone46 significantly reduced the SBE activity in the tobacco plants (Figure 13), indicating that the antibody has neutralised the enzyme activity.

### Example 10: Effect of neutralising HCV expression on GUS activity in tobacco leaves

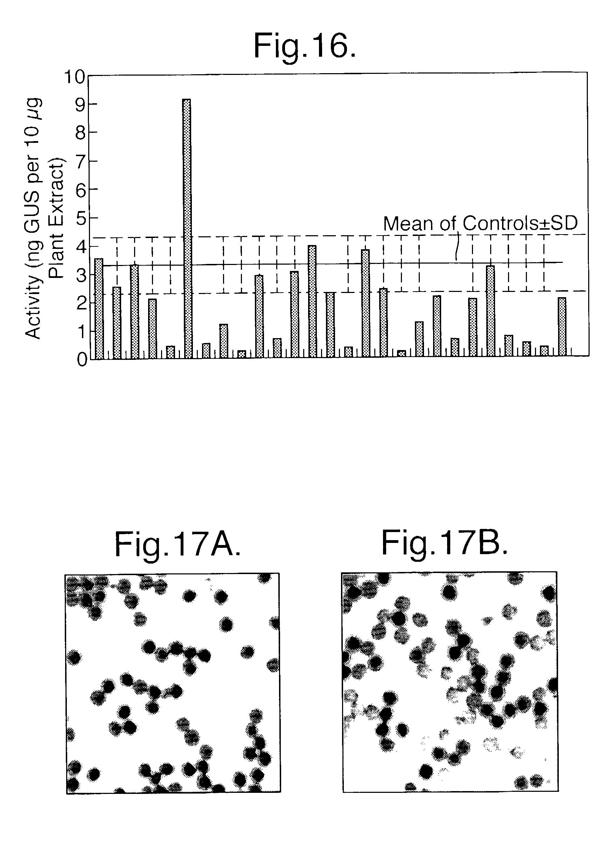

A tobacco line already transformed with expression vector pCJ.102 and shown to contain GUS activity in leaf tissue was retransformed with the expression vector pSJ35-CERV-Clone18-His6-myc-SEKDEL. Samples from 27 individual transformed plants were assayed for GUS activity in comparison to control pCJ.102 tobacco lines that had not been retransformed. A graphical representation of the results from measuring GUS activity in control lines and in lines containing Clone 18 is shown in Figure 16. A difference between the two sets of data is clearly evident, indicating that the presence of the anti-GUS antibody has decreased GUS activity.

### Example 11: Effect of neutralising HCV expression on GUS activity in tobacco pollen

The tobacco line *lat52-GUS,* containing the pollen specific promoter *lat52* driving *GUS* in a single copy homozygous state, was obtained as a gift from Dr. David Twell (Botany Department, University of Leicester, UK). This line was retransformed with expression vector pSJ.35-Lat52-Clone18-His6-myc-SEKDEL. Control lines were obtained by transforming *lat52-GUS* tobacco with pSJ.35-Lat52-HCV33-myc-SEKDEL. Pollen from 20 lines of each set of transformed tobacco were tested by histochemical staining for GUS activity. Scanned images showing the staining pattern obtained in a control line and a line containing anti-GUS VHH clone 18 are shown in Figure 17. It can quite clearly be seen that GUS activity has been 'knocked-out' by the presence of anti-GUS antibody. Half the pollen retains GUS activity (shown by blue staining) due to segregation of the transgene at meiosis.

### Example 12: Expression of whole heavy chain immunoglobulins in plants

### Construction of HCV33-IgG2b expression vectors

### pPV.5LN-HCV33-IgG2b

A cDNA clone IgG2b (Vu *et. al.,* Mol. Imm., 34, 16-17, 1997) was amplified by PCR using oligonucleotides 304 (Seq. ID. No. 58) and 305 (Seq. ID. No. 59), 306 (Seq. ID. No. 60) and 310 (Seq. ID. NO. 61) (Table 1). The amplified fragments were digested with BstEII/HincII and HincII/EcoRI respectively and inserted into NcoI/MunI opened vector pPV.5LN together with the NcoI/BstEII HCV33 fragment from vector pPV.5LN-HCV33-myc, yielding the vector pPV.5-HCV33-IgG2b. The DNA sequence of the amplified fragment was verified by sequencing with primers Seq. ID. No. 19, 53, 58, 60 and 62 (Table 1).

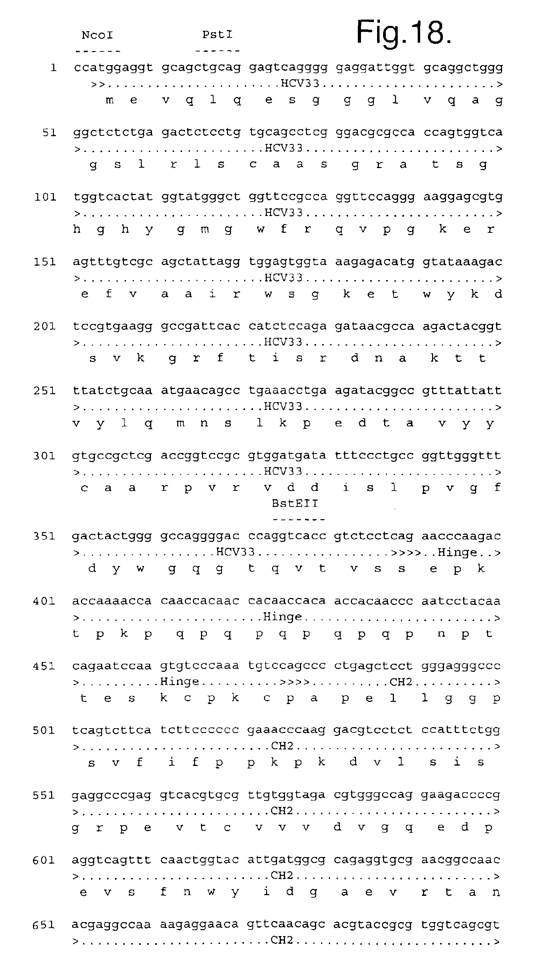

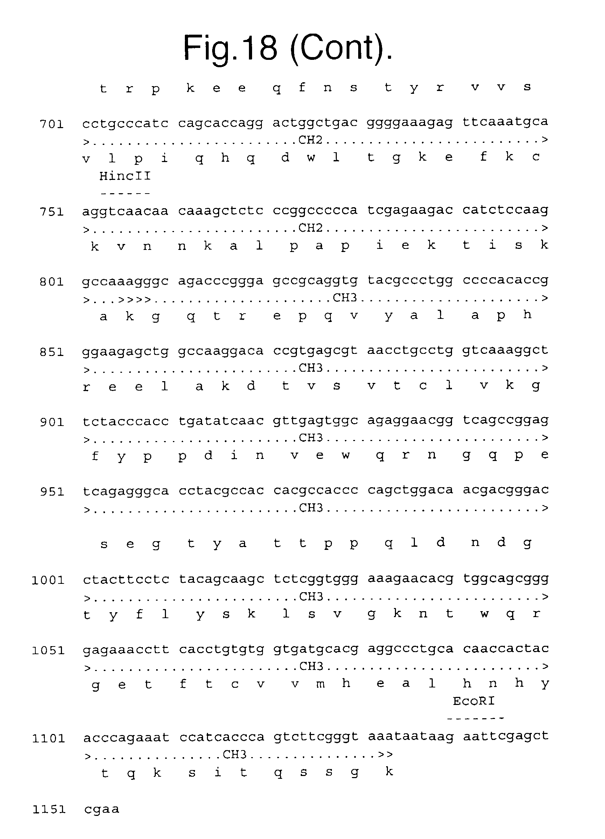

The predicted amino acid sequence contained two mutations at amino acid numbers 167 and 369 (Figure 18) as compared to the published sequence by Woolven *et. al.,* Immunogen., 50, 98-101, 1999. Oligonucleotides 314 (Seq. ID. No. 63) and 307 (Seq. ID. No. 64) (Table 1) were designed to change the mutations at amino acids 167 (proline) and 369 (Stop codon) to a serine and lysine respectively.

The HCV33-IgG2b clone was then amplified by PCR using oligonucleotides 314 and 307 (Table 1). The amplified fragment was digested with SacI/EcoRI and inserted into HindIII/MunI opened vector pPV.5LN together with the HindIII/SacI fragment from vector pPV.5-HCV33-IgG2b, yielding the vector pPV.5LN-HCV33-IgG2b. The DNA sequence of the amplified fragment was verified by sequencing with primers Seq. ID. No. No. 19, 53, 58, 60 and 62 (Table 1) (Figure 18).

### pPV.5LN-PR1a-HCV33-IgG2b

The BstEII/EcoRI HCV33-IgG2b fragment from vector pPV.5LN-HCV33-IgG2b was inserted into HindIII/EcoRI opened pPV.5LN together with the HindIII/BstEII promoter-PRla-HCV33 fragment from vector pPV.5LN-PR1a-HCV33-myc.

### pPV.5LN-GBSS-HCV33-IgG2b

The BstEII/EcoRI HCV33-IgG2b fragment from vector pPV.5LN-HCV33-IgG2b was inserted into HindIII/EcoRI opened pPV.5LN together with the HindIII/BstEII promoter-GBSS-HCV33 fragment from vector pPV.5LN-GBSS-HCV33-myc.

### pPV.5LN-PR1a-HCV33-IgG2b-SEKDEL

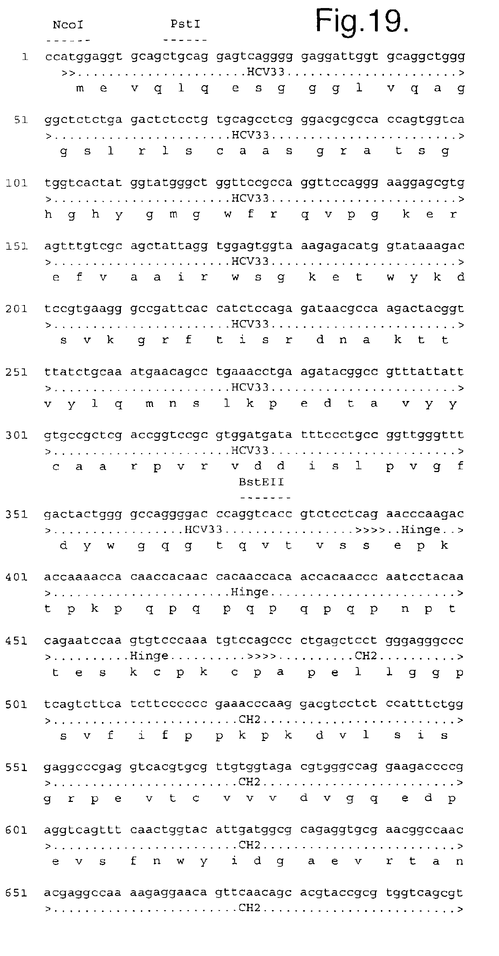

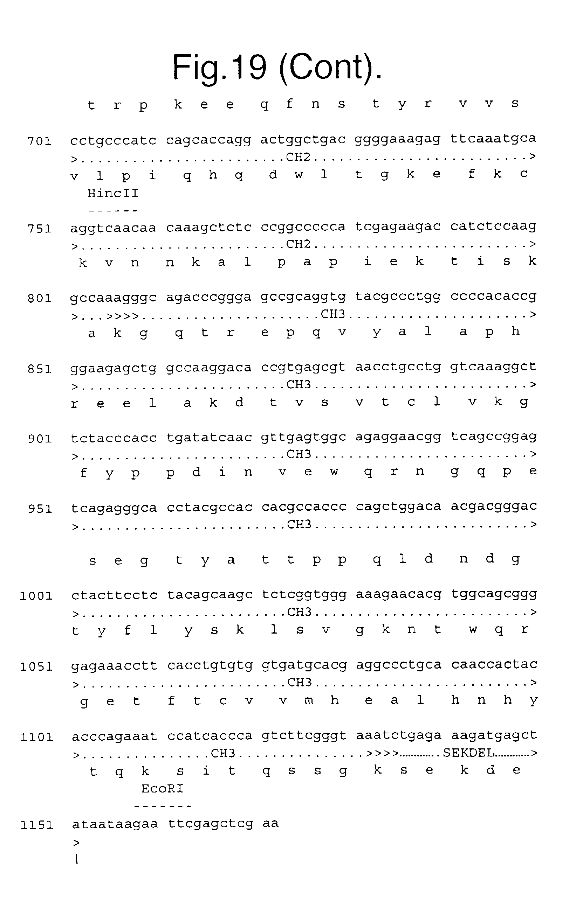

Vector pPV.5LN-HCV33-IgG2b was amplified by PCR using oligonucleotides 306 and 308 (Seq. ID. No. 65) (Table 1; to generate 3' SEKDEL endoplasmic retention signal). The amplified fragment was digested with HincII/EcoRI and inserted into HindIII/EcoRI opened pPV.5LN together with the HindIII/HincII fragment from vector pPV.5LN-PR1a-HCV33-IgG2b. The DNA sequence of the amplified fragment was the verified by sequencing with primers Seq. ID. No. 19, 63 (Table 1) (Figure 19).

### pPV.8 2x35S plant transformation vectors

The HindIII/EcoRI inserts from vectors pPV.5LN-HCV33-IgG2b, pPV.5LN-PR1a-HCV33-IgG2b, pPV.5LN-GBSS-HCV33-IgG2b and pPV.5LN-PR1a-HCV33-IgG2b-SEKDEL were inserted into HindIII/EcoRI vector fragment of pSJ.34, yielding pPV.8-HCV33-IgG2b, pPV.8-PR1a-HCV33-IgG2b, pPV.8-GBSS-HCV33-IgG2b and pPV.8-PR1a-HCV33-IgG2b-SEKDEL respectively.

### Detection of HCV33-IgG2b binding activity

As for RR6 binding activity, except:

Purified and quantified HCV33-IgG2b from tobacco plants was used as the standard.

100µl/well of 1:2,000 dilution of rabbit anti-llama IgG was used as the primary detection antibody.

100µl/well of 1ug/mL goat anti-rabbit antibody conjugated alkaline phosphatase was used as the secondary detection antibody.

### Expression of HCV33-IgG2b antigen binding activity in the cytoplasm

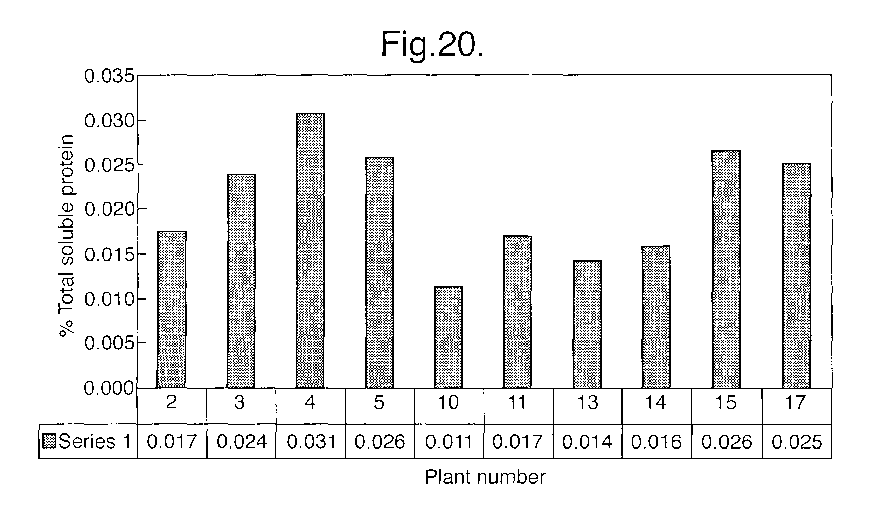

Small samples of the leaves of 20 individual Tobacco plants transformed with the expression vector pPV.8-HCV33-IgG2b were tested for the presence of anti-RR6 binding activity using ELISA. 10 of the tested samples showed low levels of RR6 binding activity. Detailed analysis of the anti-RR6 binding activity showed that HCV33-IgG2b was expressed as 0.011-0.031% of total soluble protein present (Figure 20).

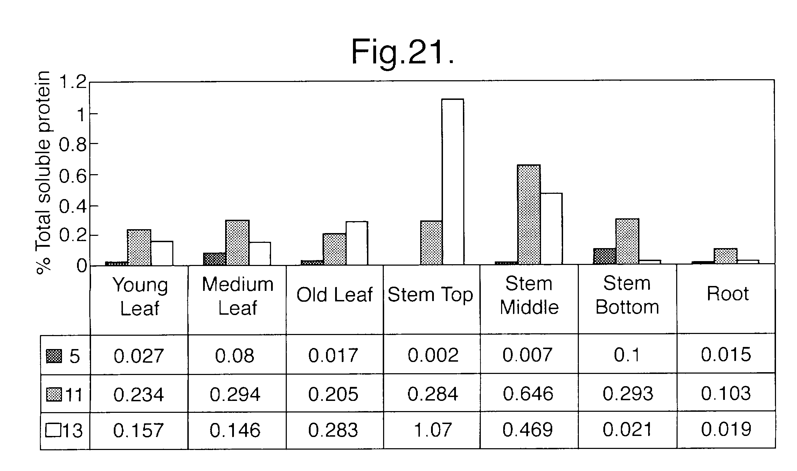

Three plants chosen at random were selfed and allowed to set seed, the next generation was germinated and after 6 weeks small samples of leaf, stem and root were tested for the presence of anti-RR6 binding activity using ELISA. Two of the tested plants showed increased levels of RR6 binding activity as compared to the original analysis. Detailed analysis of the anti-RR6 binding activity showed that HCV33-IgG2b was expressed at its highest in stem tissues at a maximum level of 1.07% total protein present (Figure 21).

### Expression of HCV33-IgG2b antigen binding activity in the plastids

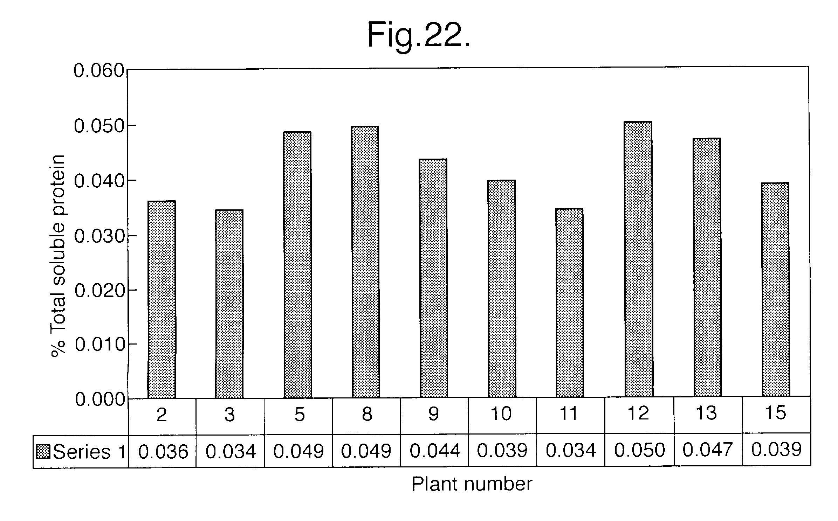

Small samples of the leaves of 20 individual Tobacco plants transformed with the expression vector pPV.8-GBSS-HCV33-IgG2b were tested for the presence of anti-RR6 binding activity using ELISA. 10 of the tested samples showed low levels of RR6 binding activity. Detailed analysis of the anti-RR6 binding activity showed that HCV33-IgG2b was expressed as 0.034-0.050% of total soluble protein present (Figure 22).

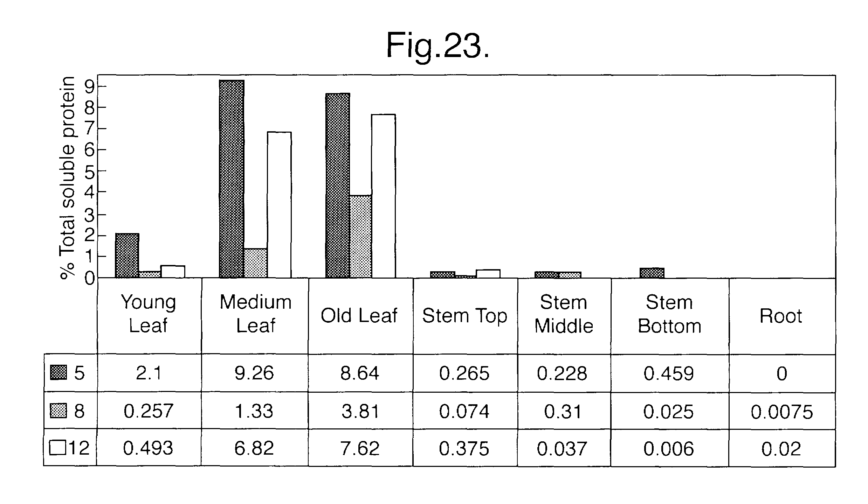

Three plants chosen at random were selfed and allowed to set seed, the next generation was germinated and after 6 weeks small samples of leaf, stem and root were tested for the presence of anti-RR6 binding activity using ELISA. All of the tested samples showed increased levels of RR6 binding activity as compared to the original analysis. Detailed analysis of the anti-RR6 binding activity showed that HCV33-IgG2b was expressed at its highest in leaf tissues at a maximum level of 9.26% total soluble protein present (Figure 23).

### Expression of HCV33-IgG2b antigen binding activity in the apoplastic space

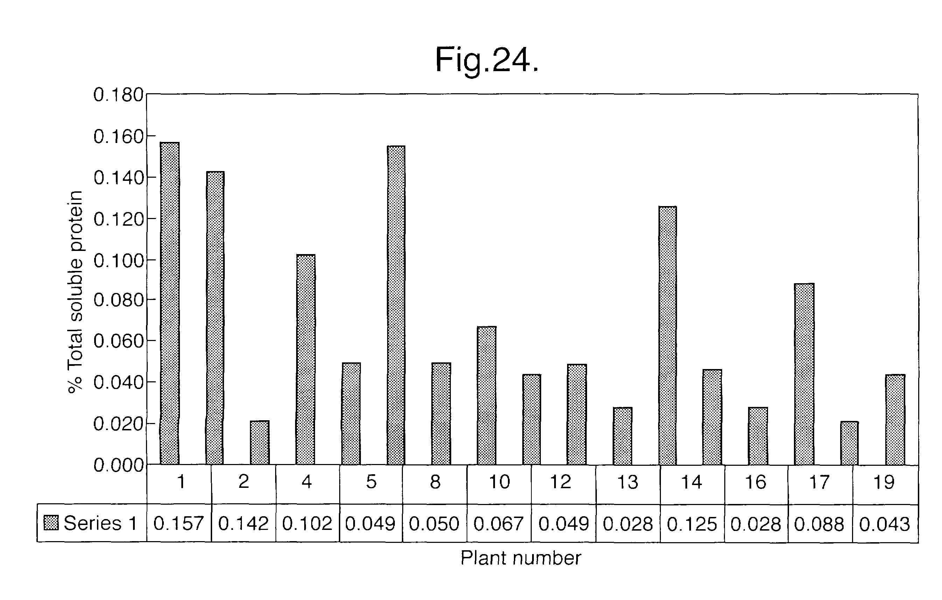

Small samples of the leaves of 20 individual Tobacco plants transformed with the expression vector pPV.8-PR1a-HCV33-IgG2b were tested for the presence of anti-RR6 binding activity using ELISA. 17 of the tested samples showed significant levels of RR6 binding activity. Detailed analysis of the anti-RR6 binding activity showed that HCV33-IgG2b was expressed as 0.021-0.157% of total soluble protein present (Figure 24).

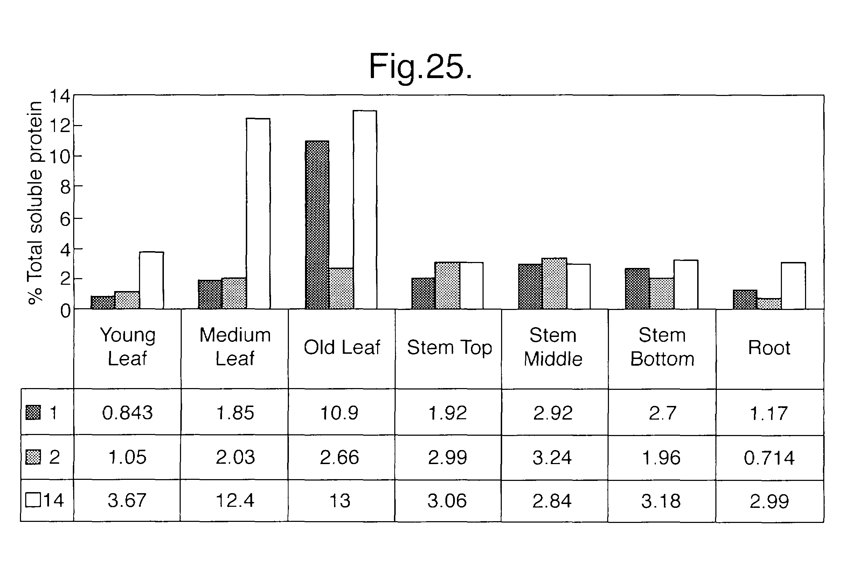

Three plants chosen at random were selfed and allowed to set seed, the next generation was germinated and after 6 weeks small samples of leaf, stem and root were tested for the presence of anti-RR6 binding activity using ELISA. All of the tested samples showed increased levels of RR6 binding activity as compared to the original analysis. Detailed analysis of the anti-RR6 binding activity showed that HCV33-IgG2b was expressed at very high levels in all tissues tested, with the highest level found in leaf at a maximum of 13% total soluble protein present (Figure 25).

### Expression of HCV33-IgG2b antigen binding activity in the endoplasmic reticulum

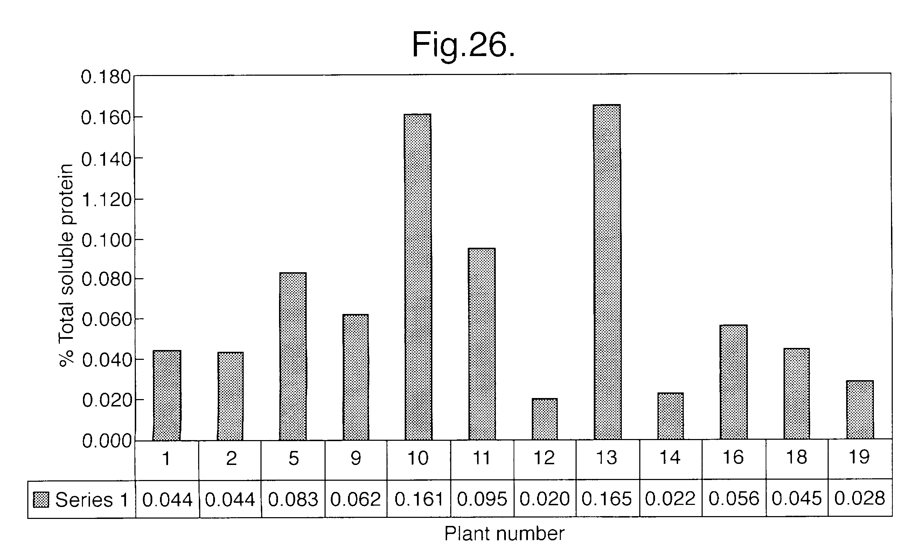

Small samples of the leaves of 20 individual Tobacco plants transformed with the expression vector pPV.8-HCV33-IgG2b were tested for the presence of anti-RR6 binding activity using ELISA. 13 of the tested samples showed significant levels of RR6 binding activity. Detailed analysis of the anti-RR6 binding activity present using showed that HCV33-IgG2b was expressed as 0.02-0.165% of total soluble protein present (Figure 26).

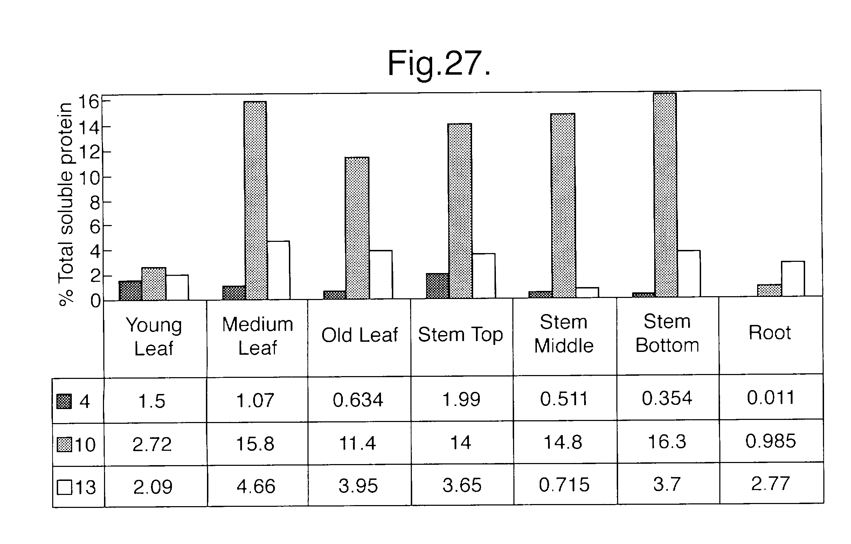

Three plants chosen at random were selfed and allowed to set seed, the next generation was germinated and after 6 weeks small samples of leaf, stem and root were tested for the presence of anti-RR6 binding activity using ELISA. All of the tested samples showed increased levels of RR6 binding activity as compared to the original analysis. Detailed analysis of the anti-RR6 binding activity showed that HCV33-IgG2b was expressed at very high levels in all tissues tested, with the highest level found in leaf at a maximum of 16.3% of total soluble protein present (Figure 27).

### Example 13: Expression of anti-SBEII (clone68) antigen binding activity in tobacco directed to the plastids

### Construction of CERV-GBSS-Clone68-myc expression vectors

### pPV.7LN-GBSS-Clone68-His6-myc

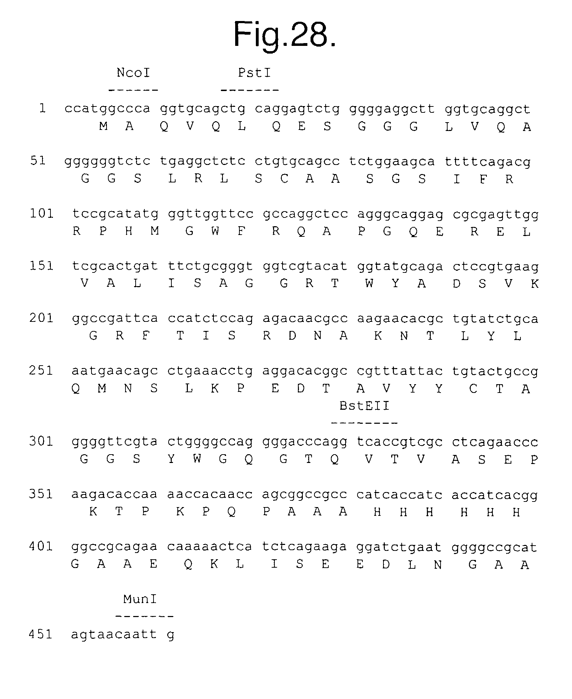

The Clone68-His6-myc sequence was amplified by PCR using oligonucleotides PCR.288 (Seq. ID. No. 47) and JWHCV2 (Seq. ID. No. 48) (Table 1) from pHEN.Clone68-His6-myc. The PstI/MunI fragment was inserted into the NcoI/MunI opened vector pPV.7LN together with a 252 bp NcoI/PstI fragment encoding the GBSS leader sequence (as described for pPV.5LN-GBSS-HCV33-myc) allowing the in-frame fusion with the 5' framework-1 sequences of the HCV fragment to create pPV.7LN-GBSS-Clone68-His6-myc. The DNA sequence of clone 68 was then verified with sequencing primers, Seq. ID. No. 19,49 (Table 1) (Figure 28).

### pSJ.35-CERV plant transformation vector

The HindIII/EcoRI inserts from vector pPV.7LN-GBSS-clone68-His6-myc were inserted into HindIII/EcoRI vector fragment of pSJ.35, yielding vector pSJ.35-CERV-GBSS-clone68-His6-myc.

### Detection of SBEII binding activity

This was performed as described in example 3 for anti-SBEII binding activity, except that purified and quantified clone68-His6-myc from *E.coli* was used as the standard.

### Expression of anti-SBEII antigen binding activity in the plastids

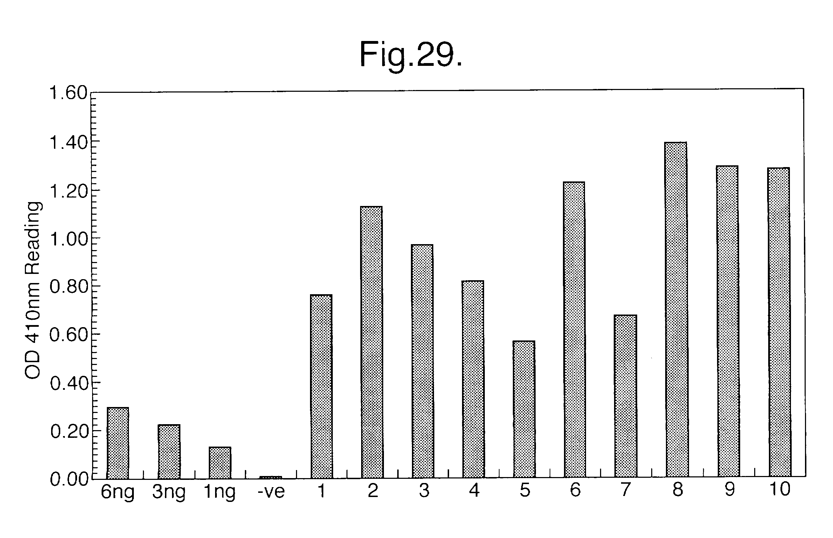

High levels of expression were obtained with another HCV antibody with anti-SBEII antigen binding specificity. Small samples of the leaves of 30 individual tobacco plants transformed with the expression vector pSJ.35-CERV-GBSS-Clone68-His6-myc were tested for the presence of anti-SBEII binding activity using ELISA. 26 of the tested plants expressed levels of SBEII-binding activity above the standards with expression levels at a minimum of 0.3% total soluble protein (Figure 29).

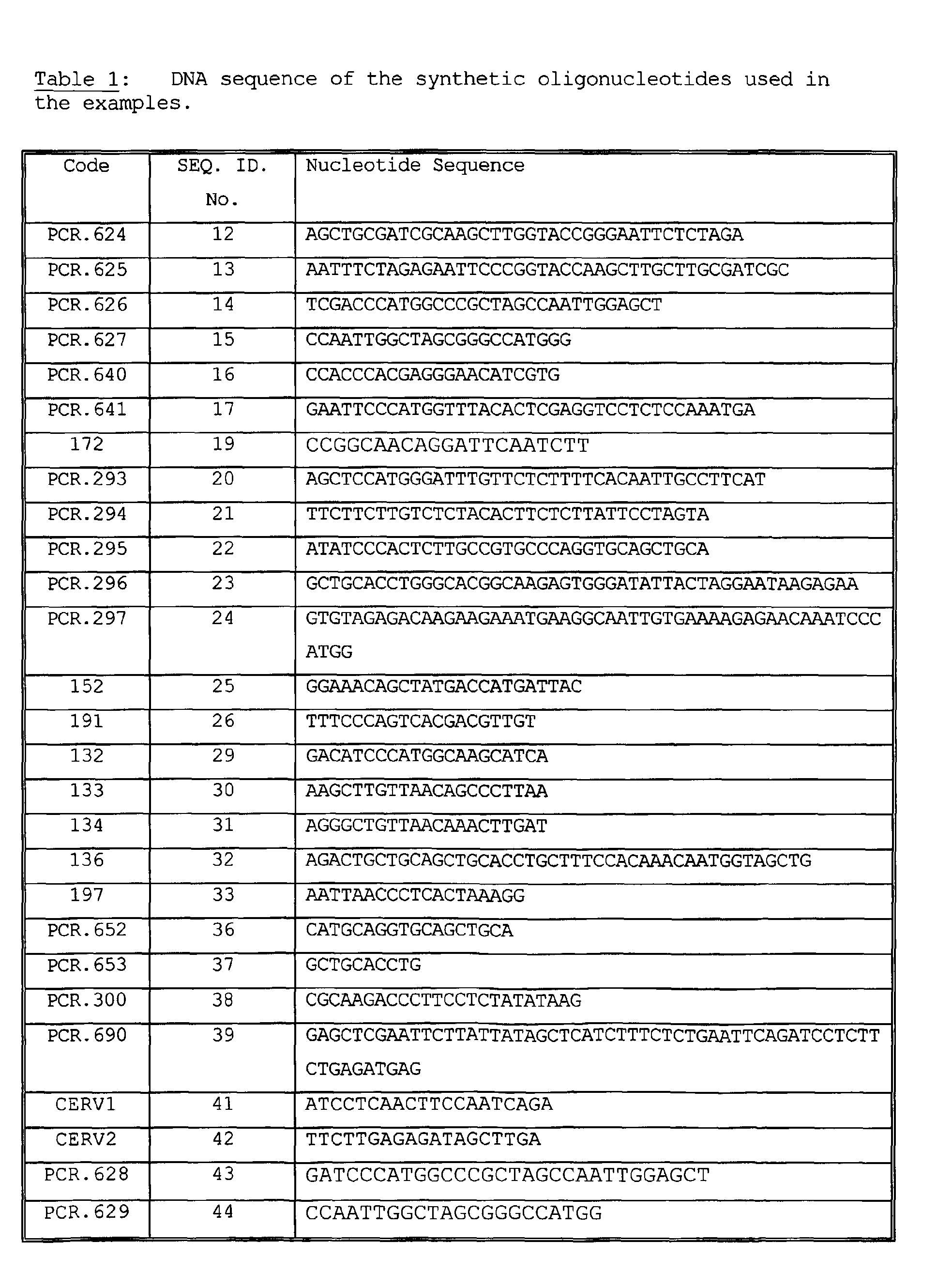

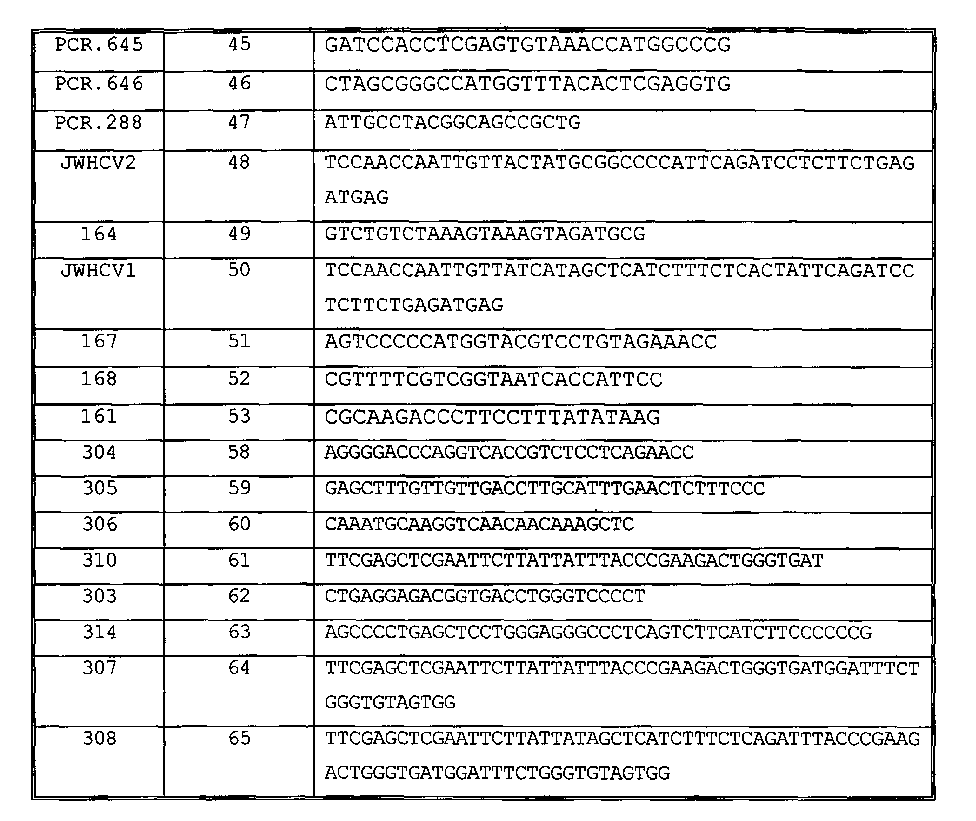

**Table 2:**

| Summary of HCV expression levels in tobacco (unless otherwise stated) detected. Maximum observed expression levels are expressed as percentage of active antibody in the total amount of soluble protein extracted. | | |
|---|---|---|
| Construct | Compartment | Expression Level |
| pPV.8-no leader-HCV33-myc | Cytosol | 0.002% |
| pPV.8-GBSS-HCV33-myc | Chloroplast | 1.0% |
| pPV.8-PRIa-HCV33-myc | Apoplast | 0.00074% |
| pPV.8-no leader-HCV33-myc-KDEL | Cytosol | <0.001% |
| pPV.8-GBSS-HCV33-myc-KDEL | Chloroplast | 0.05% |
| pPV.8-PRIa-HCV33-myc-KDEL | ER | 0.4% |
| pPV.8-GBSS-HCV33-myc | Plastid (Tomato fruit columella) | 0.2% |
| pSJ.35-CERV-GBSS-Clonel-His6-myc | Chloroplast | 0.2% |

## Claims

1. A method for modifying a plant to produce an antibody or an active fragment or derivative thereof, or a protein functionally equivalent thereto, in a desired cellular compartment comprising introducing into a plant a DNA sequence encoding a heavy chain immunoglobulin or an active fragment or derivative thereof, or a sequence encoding a protein functionally equivalent thereto, said DNA sequence being operably linked to one or more promoters and provided, as appropriate, with an additional sequence encoding a peptide sequence capable of targeting said antibody or fragment or derivative thereof to said desired cellular compartment.

2. A method according to claim 1 wherein the DNA sequence encoding the heavy chain immunoglobulin or fragment or derivative thereof is obtainable from camelids.

3. A method according to claim 1 or claim 2 wherein the plant is selected from tobacco, pea, potato, spinach, tomato or tea.

4. A method according to any one of claims 1 to 3 wherein the heavy chain immunoglobulin or active fragment or derivative thereof binds to a protein present in the plant.

5. A method according to any one of claims 1 to 3 wherein the heavy chain immunoglobulin or active fragment or derivative thereof binds to a plant or animal pathogen.

6. A method according to any one of claims 1 to 4 wherein the heavy chain immunoglobulin or active fragment or derivative thereof binds to a plant hormone or metabolite.

7. A plant prepared according to the method of any one of claims 1 to 6.

8. A modified plant having, in a desired cellular compartment, enhanced levels of heavy chain immunoglobulins or active fragments or derivatives thereof or proteins functionally equivalent thereto compared to equivalent but unmodified plants.

9. Seeds, fruits, progeny and hybrids of a plant according to claim 7 or 8.

10. A food product comprising a plant according to claim 7 or 8.

11. Use of a DNA sequence encoding a heavy chain immunoglobulin or an active fragment or derivative thereof or one or more sequences encoding a protein functionally equivalent thereto to cause a plant to produce said immunoglobulin or fragment or derivative thereof, or protein functionally equivalent thereto, in a desired cellular compartment.

12. Use according to claim 11 to increase pathogen resistance in a plant.

13. Use according to claim 11 to modulate plant metabolism.

14. A method for preparing a heavy chain immunoglobulin or an active fragment or derivative thereof comprising the steps of:
(i) modifying a plant according to the method of any of claims 1 to 6, and
(ii) extracting from said modified plant the heavy chain immunoglobulin or active fragment or derivative thereof produced therein.
